# EUROPEAN PATENT APPLICATION

(11) **EP 1 231 264 A1**
(43) Date of publication of application: **14.08.2002**
(21) Application number: 00951548.7
(22) Date of filing: 04.08.2000
(51) Int. Cl.: C12N 9/12, C12N 15/54

(54) **NEW PROTEIN**

(30) Priority: 06.08.1999 EP 99500139
(71) Applicant: Glaxo Wellcome, S.A., 28760 Tres Cantos (Madrid) (ES)
(72) Inventor: MOSCAT GUILLEN, Jorge, Centro de Biol. Molecular, 28049 Cantoblanco (ES); DIAZ-MECO CONDE, Maria Teresa Centro de Bio. Mol., 28049 Cantoblanco (ES)
(74) Representative: Stott, Michael John
(86) International application number: ES0000308
(87) International publication number: WO0111027

(57) **Abstract**

An isolated human BLIP protein or a variant thereof.

SEQUENCE LISTING

<110> Glaxo Wellcome, S.A.

<120> Protein Kinase C Interacting Protein Involved in NF-KB
      Activation

<130> PI  3718

<140>
<141>

<160> 8

<170> PatentIn Ver. 2.0

<210> 1
<211> 309
<212> PRT
<213> Homo sapiens

<400> 1
Leu Glu Leu Gln Tyr Val Ser Pro Lys Leu Leu Met Cys Arg Asp Leu
  1               5                  10                  15

Glu Leu Ala Val Pro Gly Thr Tyr Asp Pro Asn Gln Pro Ile Ile Arg
             20                  25                  30

Ile Gln Ser Ile Ala Pro Ser Leu Gln Val Ile Thr Ser Lys Gln Arg
         35                  40                  45

Pro Arg Lys Leu Thr Leu Met Gly Ser Asn Gly His Glu Phe Val Phe
     50                  55                  60

Leu Leu Lys Gly His Glu Asp Leu Arg Gln Asp Glu Arg Val Met Gln
 65                  70                  75                  80

Leu Phe Gly Leu Val Asn Thr Leu Leu Ala Asn Asp Pro Thr Ser Leu
                 85                  90                  95

Arg Lys Asn Leu Ser Ile Gln Arg Tyr Ala Val Ile Pro Leu Ser Thr
            100                 105                 110

Asn Ser Gly Leu Ile Gly Trp Val Pro His Cys Asp Thr Leu His Ala
        115                 120                 125

Leu Ile Arg Asp Tyr Arg Glu Lys Lys Lys Ile Leu Leu Asn Ile Glu
    130                 135                 140

His Arg Ile Met Leu Arg Met Ala Pro Asp Tyr Asp His Leu Thr Leu
145                 150                 155                 160

Met Gln Lys Val Glu Val Phe Glu His Ala Val Asn Asn Thr Ala Gly
                165                 170                 175

Asp Asp Leu Ala Lys Leu Leu Trp Leu Lys Ser Pro Ser Ser Glu Val
            180                 185                 190

Trp Phe Asp Arg Arg Thr Asn Tyr Thr Arg Ser Leu Ala Val Met Ser
        195                 200                 205

Met Val Gly Tyr Ile Leu Gly Leu Gly Asp Arg His Pro Ser Asn Leu
    210                 215                 220

Met Leu Asp Arg Leu Ser Gly Lys Ile Leu His Ile Asp Phe Gly Asp
225                 230                 235                 240

Cys Phe Glu Val Ala Met Thr Arg Glu Lys Phe Pro Glu Lys Ile Pro
                245                 250                 255

Phe Arg Leu Thr Arg Met Leu Thr Asn Ala Met Glu Val Thr Gly Leu
            260                 265                 270

Asp Gly Asn Tyr Arg Ile Thr Cys His Thr Val Met Glu Val Leu Arg
        275                 280                 285

Glu His Lys Asp Ser Val Met Ala Val Leu Glu Ala Phe Val Tyr Asp
    290                 295                 300

Pro Leu Leu Asn Trp
305

<210> 2
<211> 120
<212> PRT
<213> Homo sapiens

<400> 2
Pro Ala Ile Trp Phe Glu Lys Arg Leu Ala Tyr Thr Arg Ser Val Ala
1               5                   10                  15

Thr Ser Ser Ile Val Gly Tyr Ile Leu Gly Leu Gly Asp Arg His Val
            20                  25                  30

Gln Asn Ile Leu Ile Asn Glu Gln Ser Ala Glu Leu Val His Ile Asp
        35                  40                  45

Leu Gly Val Ala Phe Glu Gln Gly Lys Ile Leu Pro Thr Pro Glu Thr
    50                  55                  60

Val Pro Phe Arg Leu Thr Arg Asp Ile Val Asp Gly Met Gly Ile Thr
65                  70                  75                  80

Gly Val Glu Gly Val Phe Arg Arg Cys Cys Glu Lys Thr Met Glu Val
                85                  90                  95

Met Arg Asn Ser Gln Glu Thr Leu Leu Thr Ile Val Glu Val Leu Leu
            100                 105                 110

Tyr Asp Pro Leu Phe Asp Trp Thr
        115                 120

<210> 3
<211> 9138
<212> DNA
<213> Homo sapiens

<220>
<221> CDS
<222> (152)..(7327)

<400> 3
agaaaaaata gagtttcact ctgttgccca ggctggagtg cagtgacgca atctcggctc 60
actgcagcct ctgccctctg agttcaagtg attctcctgc ctcagcctcc tgagtagctg 120
ggattacagg tgtctgccac cgtgcccaga c atg aaa aaa ctg ctt cct aac    172
atg tta agt ccg gat ccg agg gaa ctt cag aaa tcc att gaa gtt caa    220
ttg tta aga agt tct gtt tgt ttg gca act gct tta aac ccg ata gaa    268
caa gat cag aag tgg cag tct ata act gaa aat gtg gta aag tac ttg    316
aag caa aca tcc cgc atc gct att gga cct ctg aga ctt tct act tta    364
aca gtt tca cag tct ttg cca gtt cta agt acc ttg cag ctg tat tgc 412
tca tct gct ttg gag aac aca gtt tct aac aga ctt tca aca gag gac 460
tgt ctt att cca ctc ttc agt gaa gct tta cgt tca tgt aaa cag cat 508
gac gtg agg cca tgg atg cag gca tta agg tat act atg tac cag aat 556
cag ttg ttg gag aaa att aaa gaa caa aca gtc cca att aga agc cat 604
ctc atg gaa tta ggt cta aca gca gca aaa ttt gct aga aaa cga ggg 652
aat gtg tcc ctt gca aca aga ctg ctg gca cag tgc agt gaa gtt cag 700
ctg gga aag acc acc act gca cag gat tta gtc caa cat ttt aaa aaa 748
cta tca acc caa ggt caa gtg gat gaa aaa tgg ggg ccc gaa ctt gat 796
att gaa aaa acc aaa ttg ctt tat aca gca ggc cag tca aca cat gca 844
atg gaa atg ttg agt tct tgt gcc ata tct ttc tgc aag tct gtg aaa 892
gct gaa tat gca gtt gct aaa tca att ctg aca ctg gct aaa tgg atc 940
cag gca gaa tgg aaa gag att tca gga cag ctg aaa cag gtt tac aga 988
gct cag cac caa cag aac ttc aca ggt ctt tct act ttg tct aaa aac 1036
ata ctc act cta ata gaa ctg cca tct gtt aat acg atg gaa gaa gag 1084
tat cct cgg atc gag agt gaa tct aca gtg cat att gga gtt gga gaa 1132
cct gac ttc att ttg gga cag ttg tat cac ctg tct tca gta cag gca 1180
cct gaa gta gcc aaa tct tgg gca gcg ttg gcc agc tgg gct tat agg 1228
tgg ggc aga aag gtg gtt gac aat gcc agt cag gga gaa ggt gtt cgt 1276
ctg ctg cct aga gaa aaa tct gaa gtt cag aat cta ctt cca gac act 1324
ata act gag gaa gag aaa gag aga ata tat ggt att ctt gga cag gct 1372
gtg tgt cgg ccg gcg ggg att cag gat gaa gat ata aca ctt cag ata 1420
act gag agt gaa gac aac gaa gaa gat gac atg gtt gat gtt atc tgg 1468
cgt cag ttg ata tca agc tgc cca tgg ctt tca gaa ctt gat gaa agt 1516
gca act gaa gga gtt att aaa gtg tgg agg aaa gtt gta gat aga ata 1564
ttc agc ctg tac aaa ctc tct tgc agt gca tac ttt act ttc ctt aaa     1612
ctc aac gct ggt caa att cct tta gat gag gat gac cct agg ctg cat     1660
tta agt cac aga gtg gaa cag agc act gat gac atg att gtg atg gcc     1708
aca ttg cgc ctg ctg cgg ttg ctc gtg aag cac gct ggt gag ctt cgg     1756
cag tat ctg gag cac ggc ttg gag aca aca ccc act gca cca tgg aga     1804
gga att att ccg caa ctt ttc tca cgc tta aac cac cct gaa gtg tat     1852
gtg cgc caa agt att tgt aac ctt ctc tgc cgt gtg gct caa gat tcc     1900
cca cat ctc ata ttg tat cct gca ata gtg ggt acc ata tcg ctt agt     1948
agt gaa tcc cag gct tca gga aat aaa ttt tcc act gca att cca act     1996
tta ctt ggc aat att caa gga gaa gaa ttg ctg gtt tct gaa tgt gag     2044
gga gga agt cct cct gca tct cag gat agc aat aag gat gaa cct aaa     2092
agt gga tta aat gaa gac caa gcc atg atg cag gat tgt tac agc aaa     2140
att gta gat aag ctg tcc tct gca aac ccc acc atg gta tta cag gtt 2188
cag atg ctc gtg gct gaa ctg cgc agg gtc act gtg ctc tgg gat gag 2236
ctc tgg ctg gga gtt ttg ctg caa caa cac atg tat gtc ctg aga cga 2284
att cag cag ctt gaa gat gag gtg aag aga gtc cag aac aac aac acc 2332
tta cgc aaa gaa gag aaa att gca atc atg agg gag aag cac aca gct 2380
ttg atg aag ccc atc gta ttt gct ttg gag cat gtg agg agt atc aca 2428
gcg gct cct gca gaa aca cct cat gaa aaa tgg ttt cag gat aac tat 2476
ggt gat gcc att gaa aat gcc cta gaa aaa ctg aag act cca ttg aac 2524
cct gca aag cct ggg agc agc tgg att cca ttt aaa gag ata atg cta 2572
aat ttg caa cag aga gca cag aaa cgt gca agt tac atc ttg cgt ctt 2620
gaa gaa atc agt cca tgg ttg gct gcc atg act aac act gaa att gct 2668
ctt cct ggg gaa gtc tca gcc aga gac act gtc aca atc cat agt gtg 2716
ggc gga acc atc aca atc tta ccg act aaa acc aag cca aag aaa ctt 2764
ctc ttt ctt gga tca gat ggg aag agc tat cct tat ctt ttc aaa gga 2812
ctg gag gat tta cat ctg gat gag aga ata atg cag ttc cta tct att 2860
gtg aat acc atg ttt gct aca att aat cgc caa gaa aca ccc cgg ttc 2908
cat gct cga cac tat tct gta aca cca cta gga aca aga tca gga cta 2956
atc cag tgg gta gat gga gcc aca ccc tta ttt ggt ctt tac aaa cga 3004
tgg caa caa cgg gaa gct gcc tta caa gca caa aag gcc caa gat tcc 3052
tac caa act cct cag aat cct gga att gta ccc cgt cct agt gaa ctt 3100
tat tac agt aaa att ggc cct gct ttg aaa aca gtt ggg ctt agc ctg 3148
gat gtg tcc cgt cgg gat tgg cct ctt cat gta atg aag gca gta ttg 3196
gaa gag tta atg gag gcc aca ccc ccg aat ctc ctt gcc aaa gag ctc 3244
tgg tca tct tgc aca aca cct gat gaa tgg tgg aga gtt acg cag tct 3292
tat gca aga tct act gca gtc atg tct atg gtt gga tac ata att ggc    3340
ctt gga gac aga cat ctg gat aat gtt ctt ata gat atg acg act gga    3388
gaa gtt gtt cac ata gat tac aat gtt tgc ttt gaa aaa ggt aaa agc    3436
ctt aga gtt cct gag aaa gta cct ttt cga atg aca caa aac att gaa    3484
aca gca ctg ggt gta act gga gta gaa ggt gta ttt agg ctt tca tgt    3532
gag cag gtt tta cac att atg cgg cgt ggc aga gag acc ctg ctg acg    3580
ctg ctg gag gcc ttt gtg tac gac cct ctg gtg gac tgg aca gca gga    3628
ggc gag gct ggg ttt gct ggt gct gtc tat ggt gga ggt ggc cag cag    3676
gcc gag agc aag cag agc aag aga gag atg gag cga gag atc acc cgc    3724
agc ctg ttt tct tct aga gta gct gag att aag gtg aac tgg ttt aag    3772
aat aga gat gag atg ctg gtt gtg ctt ccc aag ttg gac ggt agc tta    3820
gat gaa tac cta agc ttg caa gag caa ctg aca gat gtg gaa aaa ctg    3868
cag ggc aaa cta ctg gag gaa ata gag ttt cta gaa gga gct gaa ggg    3916
gtg gat cat cct tct cat act ctg caa cac agg tat tct gag cac acc    3964
caa cta cag act cag caa aga gct gtt cag gaa gca atc cag gtg aag    4012
ctg aat gaa ttt gaa caa tgg ata aca cat tat cag gct gca ttc aat    4060
aat tta gaa gca aca cag ctt gca agc ttg ctt caa gag ata agc aca    4108
caa atg gac ctt ggt cct cca agt tac gtg cca gca aca gcc ttt ctg    4156
cag aat gct ggt cag gcc cac ttg att agc cag tgc gag cag ctg gag    4204
ggg gag gtt ggt gct ctc ctg cag cag agg cgc tcc gtg ctc cgt ggc    4252
tgt ctg gag caa ctg cat cac tat gca acc gtg gcc ctg cag tat ccg    4300
aag gcc ata ttt cag aaa cat cga att gaa cag tgg aag acc tgg atg    4348
gaa gag ctc atc tgt aac acc aca gta gag cgt tgt caa gag ctc tat    4396
agg aaa tat gaa atg caa tat gct ccc cag cca ccc cca aca gtg tgt    4444
cag ttc atc act gcc act gaa atg acc ctg cag cga tac gca gca gac    4492
atc aac agc aga ctt att aga caa gtg gaa cgc ttg aaa cag gaa gct    4540
gtc act gtg cca gtt tgt gaa gat cag ttg aaa gaa att gaa cgt tgc    4588
att aaa gtt ttc ctt cat gag aat gga gaa gaa gga tct ttg agt cta    4636
gca agt gtt att att tct gcc ctt tgt acc ctt aca agg cgt aac ctg    4684
atg atg gaa ggt gca gcg tca agt gct gga gaa cag ctg gtt gat ctg    4732
act tct cgg gat gga gcc tgg ttc ttg gag gaa ctc tgc agt atg agc    4780
gga aac gtc acc tgc ttg gtt cag tta ctg aag cag tgc cac ctg gtg    4828
cca cag gac tta gat atc ccg aac ccc atg gaa gcg tct gag aca gtt    4876
cac tta gcc aat gga gtg tat acc tca ctt cag gaa ttg aat tcg aat    4924
ttc cgg caa atc ata ttt cca gaa gca ctt cga tgt tta atg aaa ggg    4972
gaa tac acg tta gaa agt atg ctg cat gaa ctg gac ggt ctt att gag    5020
cag acc acc gat ggc gtt ccc ctg cag act cta gtg gaa tct ctt cag    5068
gcc tac tta aga aac gca gct atg gga ctg gaa gaa gaa aca cat gct    5116
cat tac atc gat gtt gcc aga cta cta cat gct cag tac ggt gaa tta    5164
atc caa ccg aga aat ggt tca gtt gat gaa aca ccc aaa atg tca gct    5212
ggc cag atg ctt ttg gta gca ttc gat ggc atg ttt gct caa gtt gaa    5260
act gct ttc agc tta tta gtt gaa aag ttg aac aag atg gaa att ccc    5308
ata gct tgg cga aag att gac atc ata agg gaa gcc agg agt act caa    5356
gtt aat ttt ttt gat gat gat aat cac cgg cag gtg cta gaa gag att    5404
ttc ttt cta aaa aga cta cag act att aag gag ttc ttc agg ctc tgt    5452
ggt acc ttt tct aaa aca ttg tca gga tca agt tca ctt gaa gat cag    5500
aat act gtg aat ggg cct gta cag att gtc aat gtg aaa acc ctt ttt    5548
aga aac tct tgt ttc agt gaa gac caa atg gcc aaa cct atc aag gca    5596
ttc aca gct gac ttt gtg agg cag ctc ttg ata ggg cta ccc aac caa    5644
gcc ctc gga ctc aca ctg tgc agt ttt atc agt gct ctg ggt gta gac 5692
atc att gct caa gta gag gca aag gac ttt ggt gcc gaa agc aaa gtt 5740
tct gtt gat gat ctc tgt aag aaa gcg gtg gaa cat aac atc cag ata 5788
ggg aag ttc tct cag ctg gtt atg aac agg gca act gtg tta gca agt 5836
tct tac gac act gcc tgg aag aag cat gac ttg gtg cga agg cta gaa 5884
acc agt att tct tct tgt aag aca agc ctg cag cgg gtt cag ctg cat 5932
att gcc atg ttt cag tgg caa cat gaa gat cta ctt atc aat aga cca 5980
caa gcc atg tca gtc aca cct ccc cca cgg tct gct atc cta acc agc 6028
atg aaa aag aag ctg cat acc ctg agc cag att gaa act tct att gcg 6076
aca gtt cag gag aag cta gct gca ctt gaa tca agt att gaa cag cga 6124
ctc aag tgg gca ggt ggt gcc aac cct gca ttg gcc cct gta cta caa 6172
gat ttt gaa gca acg ata gct gaa aga aga aat ctt gtc ctt aaa gag 6220
agc caa aga gca agt cag gtc aca ttt ctc tgc agc aat atc att cat 6268
ttt gaa agt tta cga aca aga act gca gaa gcc tta aac ctg gat gcg 6316
gcg tta ttt gaa cta atc aag cga tgt cag cag atg tgt tcg ttt gca 6364
tca cag ttt aac agt tca gtg tct gag tta gag ctt cgt tta tta cag 6412
aga gtg gac act ggt ctt gaa cat cct att ggc agc tct gaa tgg ctt 6460
ttg tca gca cac aaa cag ttg acc cag gat atg tct act cag agg gca 6508
att cag aca gag aaa gag cag cag ata gaa acg gtc tgt gaa aca att 6556
cag aat ctg gtt gat aat ata aag act gtg ctc act ggt cat aac cga 6604
cag ctt gga gat gtc aaa cat ctc ttg aaa gct atg gct aag gat gaa 6652
gaa gct gct ctg gca gat ggt gaa gat gtt ccc tat gag aac agt gtt 6700
agg cag ttt ttg ggt gaa tat aaa tca tgg caa gac aac att caa aca 6748
gtt cta ttt aca tta gtc cag gct atg ggt cag gtt cga agt caa gaa 6796
cac gtt gaa atg ctc cag gaa atc act ccc acc ttg aaa gaa ctg aaa 6844
aca caa agt cag agt atc tat aat aat tta gtg agt ttt gca tca ccc    6892
tta gtc acc gat gca aca aat gaa tgt tcg agt cca acg tca tct gct    6940
act tat cag cca tcc ttc gct gca gca gtc cgg agt aac act ggc cag    6988
aag act cag cct gat gtc atg tca cag aat gct aga aag ctg atc cag    7036
aaa aat ctt gct aca tca gct gat act cca cca agc acc gtt cca gga    7084
act ggc aag agt gtt gct tgt agt cct aaa aag gca gtc aga gac cct    7132
aaa act ggg aaa gcg gtg caa gag aga aac tcc tat gca gtg agt gtg    7180
tgg aag aga gtg aaa gcc aag tta gag ggc cga gat gtt gat ccg aat    7228
agg agg atg tca gtt gct gaa cag gtt gac tat gtc att aag gaa gca    7276
act aat cta gat aac ttg gct cag ctg tat gaa ggt tgg aca gcc tgg    7324
gtg tgaatggcaa gacagtagat gagtctggtt aagcgaggtc agacatccac         7377
cagaatcaac tcagcctcag gcatccaaag ccacaccaca gtcggtggtg atgcaactgg 7437
gggcttactc tgaggaaacc taggaaatct cggtgcacta ggaagtgaat cccgcaggac 7497
agctgcactc agggatacgc ccaacaccat ggcctgcaac cccagggtca agggtgaagg 7557
aaagcaagct caccgcctga acacggagat tgtctttctg ccacagaaca gcagcagacg 7617
tgtcgggagg ttagctgcgg aaagaaatcg ggatgccgcg gagcacagag tgatttggaa 7677
ctccattcca cctgaccctg tgtgtacaat ccaggaaaaa aacaaacccc actcagaaac 7737
agagaaaact ggggtcgcga agaaatcaca gccaaggaag atttgatgca ttcagattct 7797
cgtgtaacac ttgttgcttg gcaacagtac tggttgggtt gaccagtaag tagaaaaagg 7857
ctaaaggcta tgcgatatga atttcagaaa tggactgaaa atggagagct atgtaacaga 7917
tacactacag tagaagaact tacttctgaa atgaagggaa aaaaaccacc ccatcgttcc 7977
ctactcctcc ccaccactta cccgttcccc ctttacctaa tctagtagat tagccatctt 8037
tcaaattcac ttttatttca gtccttatat ttcatatact tccgtctcga tgctgttaac 8097
aacttctgat aacatggaaa attcaaggat tgtttaaagg tctgatgatc acacacaaaa 8157
tgtaattccg gttatttaag tcatttctgt gattctatca tgtacagttt ccagaattgt 8217
cactgtgcat tcaaaagtaa tgaatctaac agacatttga tttaatgtac actccctttt 8277
gcttatagtg tgcatttttt ttggaggtca ttcaaatttt ccctcttctg tgatagctgt 8337
agtttctttc atagaaagta gctaatccag tgtaatcttt taccttttta aaaaccaaga 8397
tagagtatct attagagttt tacattgttg atgatagatt aacaataaag tgatgttctg 8457
gtggaggtag actgaaattt ttttaattca tgtttttcat ttgatacttt taatttacac 8517
ttagtaaatt aaaagttgtt taatttactt ggcattttag gacatgtaca tgaaacagtg 8577
aaaatgagat ccaccaacat cttttattaa gttcagttat tagtctgtga agtgctttac 8637
tttttgcaca attttaatag cttgctattc agtaatacat tatagtgaat tcatgatcaa 8697
ggtttcctta aatttagcat tgcatttcag tactgactgt gtaagctaaa ttgctgatcc 8757
aaaataaaaa cccagactag aatagggttc ttaaaatcaa gtatcaatac aaaatagaac 8817
acaattaaaa tcttaattgt tggctgggca cagtggctca cgcctgtaat cccagcactt 8877
tgggaggccg aggcgggcgg atcatgaggt taggagagcg agaccatcct ggctaacacg 8937
gtgaaacccc gtctttacta aaatacaaaa aaaattagcc gggtgtggtg gcgggcgcct 8997
gtagtcccag ctactcggga ggctgaggca ggagaatggc gtgaacccag gaggcggagc 9057
ttgcagtgag ccgagattgt gccactgcac tccagcctgg gcaacagagc tagactctgt 9117
gtcaaaaata aatgactaga t 9138

<210> 4
<211> 2392
<212> PRT
<213> Homo sapiens

<400> 4

<210> 5
<211> 2392
<212> PRT
<213> Artificial Sequence

<220>
<223> Description of Artificial Sequence: kinase
inactive BLIP protein

<400> 5
Met Lys Lys Leu Leu Pro Asn Met Leu Ser Pro Asp Pro Arg Glu Leu
1               5                   10                  15

Gln Lys Ser Ile Glu Val Gln Leu Leu Arg Ser Ser Val Cys Leu Ala
            20                  25                  30

Thr Ala Leu Asn Pro Ile Glu Gln Asp Gln Lys Trp Gln Ser Ile Thr
        35                  40                  45

Glu Asn Val Val Lys Tyr Leu Lys Gln Thr Ser Arg Ile Ala Ile Gly
    50                  55                  60

Pro Leu Arg Leu Ser Thr Leu Thr Val Ser Gln Ser Leu Pro Val Leu
65                  70                  75                  80

Ser Thr Leu Gln Leu Tyr Cys Ser Ser Ala Leu Glu Asn Thr Val Ser

                85                  90                  95

Asn Arg Leu Ser Thr Glu Asp Cys Leu Ile Pro Leu Phe Ser Glu Ala
            100                 105                 110

Leu Arg Ser Cys Lys Gln His Asp Val Arg Pro Trp Met Gln Ala Leu
        115                 120                 125

Arg Tyr Thr Met Tyr Gln Asn Gln Leu Leu Glu Lys Ile Lys Glu Gln
    130                 135                 140

Thr Val Pro Ile Arg Ser His Leu Met Glu Leu Gly Leu Thr Ala Ala
145                 150                 155                 160

Lys Phe Ala Arg Lys Arg Gly Asn Val Ser Leu Ala Thr Arg Leu Leu
                165                 170                 175

Ala Gln Cys Ser Glu Val Gln Leu Gly Lys Thr Thr Thr Ala Gln Asp
            180                 185                 190

Leu Val Gln His Phe Lys Lys Leu Ser Thr Gln Gly Gln Val Asp Glu
        195                 200                 205

Lys Trp Gly Pro Glu Leu Asp Ile Glu Lys Thr Lys Leu Leu Tyr Thr
    210                 215                 220

Ala Gly Gln Ser Thr His Ala Met Glu Met Leu Ser Ser Cys Ala Ile
225                 230                 235                 240

Ser Phe Cys Lys Ser Val Lys Ala Glu Tyr Ala Val Ala Lys Ser Ile
                245                 250                 255

Leu Thr Leu Ala Lys Trp Ile Gln Ala Glu Trp Lys Glu Ile Ser Gly
            260                 265                 270

Gln Leu Lys Gln Val Tyr Arg Ala Gln His Gln Gln Asn Phe Thr Gly
        275                 280                 285

Leu Ser Thr Leu Ser Lys Asn Ile Leu Thr Leu Ile Glu Leu Pro Ser
    290                 295                 300

Val Asn Thr Met Glu Glu Glu Tyr Pro Arg Ile Glu Ser Glu Ser Thr
305                 310                 315                 320

Val His Ile Gly Val Gly Glu Pro Asp Phe Ile Leu Gly Gln Leu Tyr
                325                 330                 335

His Leu Ser Ser Val Gln Ala Pro Glu Val Ala Lys Ser Trp Ala Ala
            340                 345                 350

Leu Ala Ser Trp Ala Tyr Arg Trp Gly Arg Lys Val Val Asp Asn Ala
        355                 360                 365

Ser Gln Gly Glu Gly Val Arg Leu Leu Pro Arg Glu Lys Ser Glu Val
    370                 375                 380

Gln Asn Leu Leu Pro Asp Thr Ile Thr Glu Glu Glu Lys Glu Arg Ile
385                 390                 395                 400

Tyr Gly Ile Leu Gly Gln Ala Val Cys Arg Pro Ala Gly Ile Gln Asp
                405                 410                 415

Glu Asp Ile Thr Leu Gln Ile Thr Glu Ser Glu Asp Asn Glu Glu Asp
            420                 425                 430

Asp Met Val Asp Val Ile Trp Arg Gln Leu Ile Ser Ser Cys Pro Trp
        435                 440                 445

Leu Ser Glu Leu Asp Glu Ser Ala Thr Glu Gly Val Ile Lys Val Trp
    450                 455                 460

Arg Lys Val Val Asp Arg Ile Phe Ser Leu Tyr Lys Leu Ser Cys Ser
465                 470                 475                 480

Ala Tyr Phe Thr Phe Leu Lys Leu Asn Ala Gly Gln Ile Pro Leu Asp
                485                 490                 495

Glu Asp Asp Pro Arg Leu His Leu Ser His Arg Val Glu Gln Ser Thr
            500                 505                 510

Asp Asp Met Ile Val Met Ala Thr Leu Arg Leu Leu Arg Leu Leu Val
        515                 520                 525

Lys His Ala Gly Glu Leu Arg Gln Tyr Leu Glu His Gly Leu Glu Thr
    530                 535                 540

Thr Pro Thr Ala Pro Trp Arg Gly Ile Ile Pro Gln Leu Phe Ser Arg
545                 550                 555                 560

Leu Asn His Pro Glu Val Tyr Val Arg Gln Ser Ile Cys Asn Leu Leu
                565                 570                 575

Cys Arg Val Ala Gln Asp Ser Pro His Leu Ile Leu Tyr Pro Ala Ile
            580                 585                 590

Val Gly Thr Ile Ser Leu Ser Ser Glu Ser Gln Ala Ser Gly Asn Lys
        595                 600                 605
Phe Ser Thr Ala Ile Pro Thr Leu Leu Gly Asn Ile Gln Gly Glu Glu

610                 615                 620

Leu Leu Val Ser Glu Cys Glu Gly Gly Ser Pro Pro Ala Ser Gln Asp
625                 630                 635                 640

Ser Asn Lys Asp Glu Pro Lys Ser Gly Leu Asn Glu Asp Gln Ala Met
                645                 650                 655

Met Gln Asp Cys Tyr Ser Lys Ile Val Asp Lys Leu Ser Ser Ala Asn
            660                 665                 670

Pro Thr Met Val Leu Gln Val Gln Met Leu Val Ala Glu Leu Arg Arg
        675                 680                 685

Val Thr Val Leu Trp Asp Glu Leu Trp Leu Gly Val Leu Leu Gln Gln
    690                 695                 700

His Met Tyr Val Leu Arg Arg Ile Gln Gln Leu Glu Asp Glu Val Lys
705                 710                 715                 720

Arg Val Gln Asn Asn Asn Thr Leu Arg Lys Glu Glu Lys Ile Ala Ile
                725                 730                 735

Met Arg Glu Lys His Thr Ala Leu Met Lys Pro Ile Val Phe Ala Leu
            740                 745                 750

Glu His Val Arg Ser Ile Thr Ala Ala Pro Ala Glu Thr Pro His Glu
        755                 760                 765

Lys Trp Phe Gln Asp Asn Tyr Gly Asp Ala Ile Glu Asn Ala Leu Glu
    770                 775                 780

Lys Leu Lys Thr Pro Leu Asn Pro Ala Lys Pro Gly Ser Ser Trp Ile
785                 790                 795                 800

Pro Phe Lys Glu Ile Met Leu Asn Leu Gln Gln Arg Ala Gln Lys Arg
                805                 810                 815

Ala Ser Tyr Ile Leu Arg Leu Glu Glu Ile Ser Pro Trp Leu Ala Ala
            820                 825                 830

Met Thr Asn Thr Glu Ile Ala Leu Pro Gly Glu Val Ser Ala Arg Asp
        835                 840                 845

Thr Val Thr Ile His Ser Val Gly Gly Thr Ile Thr Ile Leu Pro Thr
    850                 855                 860

Lys Thr Lys Pro Lys Lys Leu Leu Phe Leu Gly Ser Asp Gly Lys Ser
865                 870                 875                 880

Tyr Pro Tyr Leu Phe Lys Gly Leu Glu Asp Leu His Leu Asp Glu Arg
                885                 890                 895

Ile Met Gln Phe Leu Ser Ile Val Asn Thr Met Phe Ala Thr Ile Asn
            900                 905                 910

Arg Gln Glu Thr Pro Arg Phe His Ala Arg His Tyr Ser Val Thr Pro
        915                 920                 925

Leu Gly Thr Arg Ser Gly Leu Ile Gln Trp Val Asp Gly Ala Thr Pro
    930                 935                 940

Leu Phe Gly Leu Tyr Lys Arg Trp Gln Gln Arg Glu Ala Ala Leu Gln
945                 950                 955                 960

Ala Gln Lys Ala Gln Asp Ser Tyr Gln Thr Pro Gln Asn Pro Gly Ile
                965                 970                 975

Val Pro Arg Pro Ser Glu Leu Tyr Tyr Ser Lys Ile Gly Pro Ala Leu
            980                 985                 990

Lys Thr Val Gly Leu Ser Leu Asp Val Ser Arg Arg Asp Trp Pro Leu
        995                 1000                1005

His Val Met Lys Ala Val Leu Glu Glu Leu Met Glu Ala Thr Pro Pro
    1010                1015                1020

Asn Leu Leu Ala Lys Glu Leu Trp Ser Ser Cys Thr Thr Pro Asp Glu
1025                1030                1035                1040

Trp Trp Arg Val Thr Gln Ser Tyr Ala Arg Ser Thr Ala Val Met Ser
                1045                1050                1055

Met Val Gly Tyr Ile Ile Gly Leu Gly Ala Arg His Leu Asp Lys Val
            1060                1065                1070

Leu Ile Asp Met Thr Thr Gly Glu Val Val His Ile Asp Tyr Asn Val
        1075                1080                1085

Cys Phe Glu Lys Gly Lys Ser Leu Arg Val Pro Glu Lys Val Pro Phe
    1090                1095                1100

Arg Met Thr Gln Asn Ile Glu Thr Ala Leu Gly Val Thr Gly Val Glu
1105                1110                1115                1120

Gly Val Phe Arg Leu Ser Cys Glu Gln Val Leu His Ile Met Arg Arg
                1125                1130                1135

Gly Arg Glu Thr Leu Leu Thr Leu Leu Glu Ala Phe Val Tyr Asp Pro
        1140                1145                1150

Leu Val Asp Trp Thr Ala Gly Gly Glu Ala Gly Phe Ala Gly Ala Val
    1155                1160                1165

Tyr Gly Gly Gly Gly Gln Gln Ala Glu Ser Lys Gln Ser Lys Arg Glu
1170                1175                1180

Met Glu Arg Glu Ile Thr Arg Ser Leu Phe Ser Ser Arg Val Ala Glu
1185                1190                1195                1200

Ile Lys Val Asn Trp Phe Lys Asn Arg Asp Glu Met Leu Val Val Leu
                1205                1210                1215

Pro Lys Leu Asp Gly Ser Leu Asp Glu Tyr Leu Ser Leu Gln Glu Gln
            1220                1225                1230

Leu Thr Asp Val Glu Lys Leu Gln Gly Lys Leu Leu Glu Glu Ile Glu
        1235                1240                1245

Phe Leu Glu Gly Ala Glu Gly Val Asp His Pro Ser His Thr Leu Gln
    1250                1255                1260

His Arg Tyr Ser Glu His Thr Gln Leu Gln Thr Gln Gln Arg Ala Val
1265                1270                1275                1280

Gln Glu Ala Ile Gln Val Lys Leu Asn Glu Phe Glu Gln Trp Ile Thr
                1285                1290                1295

His Tyr Gln Ala Ala Phe Asn Asn Leu Glu Ala Thr Gln Leu Ala Ser
            1300                1305                1310

Leu Leu Gln Glu Ile Ser Thr Gln Met Asp Leu Gly Pro Pro Ser Tyr
        1315                1320                1325

Val Pro Ala Thr Ala Phe Leu Gln Asn Ala Gly Gln Ala His Leu Ile
    1330                1335                1340

Ser Gln Cys Glu Gln Leu Glu Gly Glu Val Gly Ala Leu Leu Gln Gln
1345                1350                1355                1360

Arg Arg Ser Val Leu Arg Gly Cys Leu Glu Gln Leu His His Tyr Ala
                1365                1370                1375

Thr Val Ala Leu Gln Tyr Pro Lys Ala Ile Phe Gln Lys His Arg Ile
            1380                1385                1390

Glu Gln Trp Lys Thr Trp Met Glu Glu Leu Ile Cys Asn Thr Thr Val

1395                1400                1405

Glu Arg Cys Gln Glu Leu Tyr Arg Lys Tyr Glu Met Gln Tyr Ala Pro
    1410                1415                1420

Gln Pro Pro Pro Thr Val Cys Gln Phe Ile Thr Ala Thr Glu Met Thr
1425                1430                1435                1440

Leu Gln Arg Tyr Ala Ala Asp Ile Asn Ser Arg Leu Ile Arg Gln Val
                1445                1450                1455

Glu Arg Leu Lys Gln Glu Ala Val Thr Val Pro Val Cys Glu Asp Gln
            1460                1465                1470

Leu Lys Glu Ile Glu Arg Cys Ile Lys Val Phe Leu His Glu Asn Gly
        1475                1480                1485

Glu Glu Gly Ser Leu Ser Leu Ala Ser Val Ile Ile Ser Ala Leu Cys
    1490                1495                1500

Thr Leu Thr Arg Arg Asn Leu Met Met Glu Gly Ala Ala Ser Ser Ala
1505                1510                1515                1520

Gly Glu Gln Leu Val Asp Leu Thr Ser Arg Asp Gly Ala Trp Phe Leu
                1525                1530                1535

Glu Glu Leu Cys Ser Met Ser Gly Asn Val Thr Cys Leu Val Gln Leu
            1540                1545                1550

Leu Lys Gln Cys His Leu Val Pro Gln Asp Leu Asp Ile Pro Asn Pro
        1555                1560                1565

Met Glu Ala Ser Glu Thr Val His Leu Ala Asn Gly Val Tyr Thr Ser
    1570                1575                1580

Leu Gln Glu Leu Asn Ser Asn Phe Arg Gln Ile Ile Phe Pro Glu Ala
1585                1590                1595                1600

Leu Arg Cys Leu Met Lys Gly Glu Tyr Thr Leu Glu Ser Met Leu His
                1605                1610                1615

Glu Leu Asp Gly Leu Ile Glu Gln Thr Thr Asp Gly Val Pro Leu Gln
            1620                1625                1630

Thr Leu Val Glu Ser Leu Gln Ala Tyr Leu Arg Asn Ala Ala Met Gly
        1635                1640                1645

Leu Glu Glu Glu Thr His Ala His Tyr Ile Asp Val Ala Arg Leu Leu
    1650                1655                1660

His Ala Gln Tyr Gly Glu Leu Ile Gln Pro Arg Asn Gly Ser Val Asp
1665                1670                1675                1680

Glu Thr Pro Lys Met Ser Ala Gly Gln Met Leu Leu Val Ala Phe Asp
                1685                1690                1695

Gly Met Phe Ala Gln Val Glu Thr Ala Phe Ser Leu Leu Val Glu Lys
            1700                1705                1710

Leu Asn Lys Met Glu Ile Pro Ile Ala Trp Arg Lys Ile Asp Ile Ile
        1715                1720                1725

Arg Glu Ala Arg Ser Thr Gln Val Asn Phe Phe Asp Asp Asn His
    1730                1735                1740

Arg Gln Val Leu Glu Glu Ile Phe Phe Leu Lys Arg Leu Gln Thr Ile
1745                1750                1755                1760

Lys Glu Phe Phe Arg Leu Cys Gly Thr Phe Ser Lys Thr Leu Ser Gly
                1765                1770                1775

Ser Ser Ser Leu Glu Asp Gln Asn Thr Val Asn Gly Pro Val Gln Ile
            1780                1785                1790

Val Asn Val Lys Thr Leu Phe Arg Asn Ser Cys Phe Ser Glu Asp Gln
        1795                1800                1805

Met Ala Lys Pro Ile Lys Ala Phe Thr Ala Asp Phe Val Arg Gln Leu
    1810                1815                1820

Leu Ile Gly Leu Pro Asn Gln Ala Leu Gly Leu Thr Leu Cys Ser Phe
1825                1830                1835                1840

Ile Ser Ala Leu Gly Val Asp Ile Ile Ala Gln Val Glu Ala Lys Asp
                1845                1850                1855

Phe Gly Ala Glu Ser Lys Val Ser Val Asp Asp Leu Cys Lys Lys Ala
            1860                1865                1870

Val Glu His Asn Ile Gln Ile Gly Lys Phe Ser Gln Leu Val Met Asn
        1875                1880                1885

Arg Ala Thr Val Leu Ala Ser Ser Tyr Asp Thr Ala Trp Lys Lys His
    1890                1895                1900

Asp Leu Val Arg Arg Leu Glu Thr Ser Ile Ser Ser Cys Lys Thr Ser
1905                1910                1915                1920

Leu Gln Arg Val Gln Leu His Ile Ala Met Phe Gln Trp Gln His Glu
1925 1930 1935

Asp Leu Leu Ile Asn Arg Pro Gln Ala Met Ser Val Thr Pro Pro Pro
                1940                1945                1950

Arg Ser Ala Ile Leu Thr Ser Met Lys Lys Lys Leu His Thr Leu Ser
            1955                1960                1965

Gln Ile Glu Thr Ser Ile Ala Thr Val Gln Glu Lys Leu Ala Ala Leu
        1970                1975                1980

Glu Ser Ser Ile Glu Gln Arg Leu Lys Trp Ala Gly Gly Ala Asn Pro
1985 1990 1995 2000

Ala Leu Ala Pro Val Leu Gln Asp Phe Glu Ala Thr Ile Ala Glu Arg
2005 2010 2015

Arg Asn Leu Val Leu Lys Glu Ser Gln Arg Ala Ser Gln Val Thr Phe
                2020                2025                2030

Leu Cys Ser Asn Ile Ile His Phe Glu Ser Leu Arg Thr Arg Thr Ala
            2035                2040                2045

Glu Ala Leu Asn Leu Asp Ala Ala Leu Phe Glu Leu Ile Lys Arg Cys
        2050                2055                2060

Gln Gln Met Cys Ser Phe Ala Ser Gln Phe Asn Ser Ser Val Ser Glu
2065 2070 2075 2080

Leu Glu Leu Arg Leu Leu Gln Arg Val Asp Thr Gly Leu Glu His Pro
2085 2090 2095

Ile Gly Ser Ser Glu Trp Leu Leu Ser Ala His Lys Gln Leu Thr Gln
                2100                2105                2110

Asp Met Ser Thr Gln Arg Ala Ile Gln Thr Glu Lys Glu Gln Gln Ile
            2115                2120                2125

Glu Thr Val Cys Glu Thr Ile Gln Asn Leu Val Asp Asn Ile Lys Thr
        2130                2135                2140

Val Leu Thr Gly His Asn Arg Gln Leu Gly Asp Val Lys His Leu Leu
2145 2150 2155 2160

Lys Ala Met Ala Lys Asp Glu Glu Ala Ala Leu Ala Asp Gly Glu Asp
2165 2170 2175
Val Pro Tyr Glu Asn Ser Val Arg Gln Phe Leu Gly Glu Tyr Lys Ser
                2180                2185                2190

Trp Gln Asp Asn Ile Gln Thr Val Leu Phe Thr Leu Val Gln Ala Met
            2195                2200                2205

Gly Gln Val Arg Ser Gln Glu His Val Glu Met Leu Gln Glu Ile Thr
        2210                2215                2220

Pro Thr Leu Lys Glu Leu Lys Thr Gln Ser Gln Ser Ile Tyr Asn Asn
2225 2230 2235 2240

Leu Val Ser Phe Ala Ser Pro Leu Val Thr Asp Ala Thr Asn Glu Cys
2245 2250 2255

Ser Ser Pro Thr Ser Ser Ala Thr Tyr Gln Pro Ser Phe Ala Ala Ala
                2260                2265                2270

Val Arg Ser Asn Thr Gly Gln Lys Thr Gln Pro Asp Val Met Ser Gln
            2275                2280                2285

Asn Ala Arg Lys Leu Ile Gln Lys Asn Leu Ala Thr Ser Ala Asp Thr
        2290                2295                2300

Pro Pro Ser Thr Val Pro Gly Thr Gly Lys Ser Val Ala Cys Ser Pro
2305 2310 2315 2320

Lys Lys Ala Val Arg Asp Pro Lys Thr Gly Lys Ala Val Gln Glu Arg
2325 2330 2335

Asn Ser Tyr Ala Val Ser Val Trp Lys Arg Val Lys Ala Lys Leu Glu
                2340                2345                2350

Gly Arg Asp Val Asp Pro Asn Arg Arg Met Ser Val Ala Glu Gln Val
            2355                2360                2365

Asp Tyr Val Ile Lys Glu Ala Thr Asn Leu Asp Asn Leu Ala Gln Leu
        2370                2375                2380

Tyr Glu Gly Trp Thr Ala Trp Val
    2385                2390

<210> 6
<211> 779
<212> PRT
<213> Homo sapiens

<400> 6
Met Leu His Glu Leu Asp Gly Leu Ile Glu Gln Thr Thr Asp Gly Val
1               5                   10                  15

## Description

### Field of the Invention

The present invention relates to human lambda interacting proteins involved in Nuclear Factor κB (NF-κB) activation and also, but not exclusively, to kinase-inactive mutants of such proteins; nucleotide sequences encoding such proteins and mutant proteins; and expression vectors, cell lines, antibodies, screening methods, compounds, methods of production and methods of treatment related to the proteins.

### Background of the Invention

The atypical Protein Kinase C (PKC) isotypes (λ/ 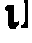 PKC and ζPKC) are necessary for basic cellular functions such as proliferation and survival. Studies have demonstrated that the atypical PKCs (aPKCs) are stimulated by TNFα and are required for the activation of NF-κB by this cytokine, through a mechanism that likely involves the phosphorylation of IκB through the activation of IκB kinase β.

The transcription factor NF-κB plays a critical role in a number of cell functions including key inflammatory and immune responses. Nuclear factor-κB is a ubiquitously expressed multisubunit transcription factor activated in several cell types by a diverse group of inflammatory agents such as TNFα, IL-1β, bacterial endotoxin, and RNA viruses. NF-κB is composed of dimers of different members of the Rel protein family. The classical form of NF-κB is an heterodimer of p50 and p65 (Rel A) (Baeurle and Henkel, 1994; Baldwin, 1996; Thanos and Maniatis, 1995); this is sequestered in the cytosol by IκB which prevents its nuclear translocation and activity.

Upon cell stimulation by inflammatory cytokines such as TNFα or IL-1, IκBα is phosphorylated, triggering the ubiquitination and subsequent degradation of IκB through the proteosome pathway (Verma et al., 1995). These events release NF-κB which translocates to the nucleus where it activates several genes (Baeurle and Henkel, 1994; Baldwin, 1996; Thanos and Maniatis, 1995; Verma et al., 1995).

The identification of the kinase responsible for the signal-induced phosphorylation of IκB has been a matter of research. Several groups have identified and cloned two IκB kinase activities (IKKα and IKKβ) that phosphorylate residues 32 and 36 of IκBα, and whose activity is potently stimulated by TNF and IL-1 (DiDonato et al., 1997; Mercurio et al., 1997; Réginer et al., 1997; Woronicz et al., 1997; Zandi et al., 1997). The IKKs bind NIK (NFκB-inducing kinase), a member of the MAPKKK family that interacts with TRAF2 (Malinin et al., 1997), thus linking IκB degradation and NF-κB activation to the TNF receptor complex.

TNFα is a potent activator of the atypical PKCs in vivo. It has previously been shown that the atypical PKC isoforms ζ (zeta) and λ/ 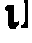 (lambda/iota) play a critical role during NF-κB activation (Diaz-Meco et al., 1996; Folgueira et al., 1996; Sontag et al., 1997). Thus, the blockade of the atypical PKCs by either microinjected pseudosubstrate peptide inhibitors (Dominguez et al., 1993), antisense oligonucleotides (Folgueira et al., 1996), or the transfection of kinase-dead dominant negative mutants of ζPKC or λ/ 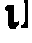 PKC (Berra et al., 1995; Bjorkoy et al., 1995; Diaz-Meco et al., 1996; Sontag et al., 1997), dramatically impairs NF-κB activation.

However, the mechanisms whereby the atypical PKCs participate in the NF-κB activation pathway remained unclear. Because ζPKC is unable to directly phosphorylate IκB (Diaz-Meco et al., 1994), it is possible that the signals generated by the stimulation of the atypical PKCs could be mediated by the novel IKKs. In this regard, it has recently been demonstrated that the atypical PKCs bind to the IKKs *in vitro* and *in vivo* (Lallena et al., 1999). Overexpression of these PKCs positively modulates IKKβ but not IKKα activity, whereas the transfection of their respective dominant negative mutants severely impairs the activation of IKKβ but not of IKKα in TNFα-stimulated cells (Lallena et al., 1999). In addition, recombinant active atypical PKC dramatically stimulates *in vitro* the IKKβ -- but not IKKα -- activity from unstimulated cells (Lallena et al., 1999). Collectively these results demonstrate a critical role of the atypical PKCs in the NF-κB pathway through the regulation of IKKβ activity.

In contrast to the classical or novel isoforms of PKCs, the atypical PKCs are insensitive to phorbol esters or diacyglycerol, but are activated by other important lipid second messengers such as phosphatidylinositol 3,4,5-P₃ (Nakanishi et al.,1993), and ceramide (Lozano et al., 1994). Although the products of PI 3-kinase activate the aPKCs (Nakanishi et al., 1993; Akimoto et al., 1996), they also stimulate other kinases such as εPKC, θPKC, PRK1 or c-Akt (Diaz-Meco et al., 1996). Ceramide activates both aPKCs (Lozano et al., 1994; Müller et al., 1995) but also stimulates the ceramide-activated protein kinase, among other possible targets. This suggests that the different lipid mediators are not selective for a given PKC.

It has now been discovered that a novel lambda interacting protein (termed BLIP herein) interacts with λ/ 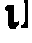 PKC in a TNFα-dependent manner. The ectopic expression of wild-type BLIP is sufficient to activate NF-κB in a λ/ 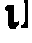 PKC dependent manner, whereas a BLIP kinase-inactive mutant is dominant negative. Human and other mammalian BLIP proteins are useful in screening methods for the identification and development of novel pharmaceutical agents, including agonists and antagonists of the proteins, which may be used to increase or decrease the activation of NFκB (compared to activation which would occur in an untreated control cell).

Accordingly, it is an object of the present invention to provide . isolated human BLIP proteins and DNA encoding such proteins. Other objects of the present invention will become apparent from the following detailed description thereof.

### Summary of the Invention

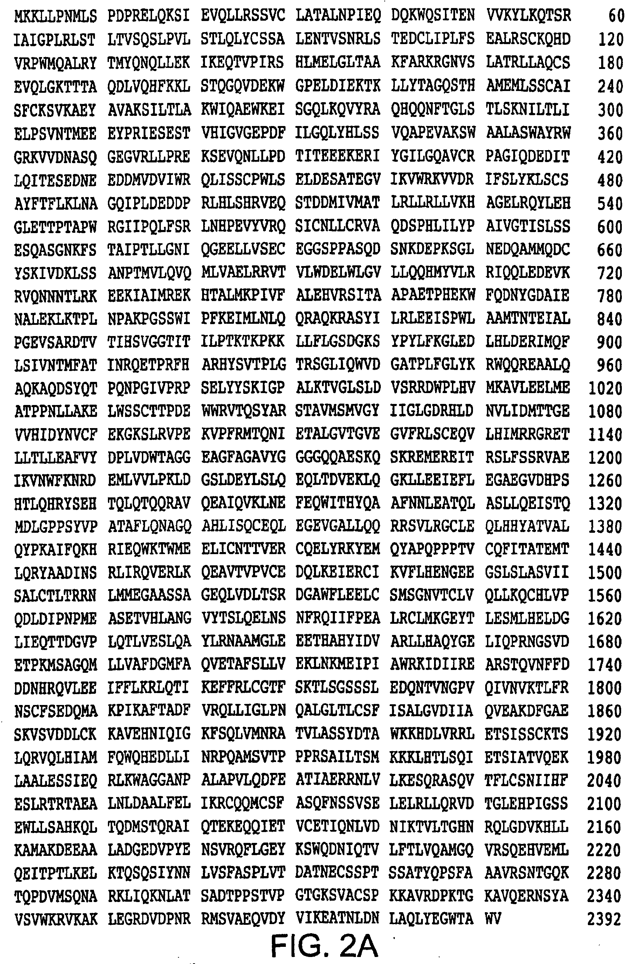

According to one embodiment of the present invention there is provided an isolated human BLIP protein or a variant thereof. In a particularly preferred aspect of the invention the protein has an amino acid sequence as provided in **Figure 2A** (SEQ ID NO:4).

A further aspect of the present invention is a kinase-dead mutant of a BLIP protein.

A further aspect of the present invention is an isolated human LIP protein or a variant thereof. In a particularly preferred aspect of the invention the protein has an amino acid sequence of SEQ ID NO:6.

According to another aspect of the invention there is provided an isolated nucleotide sequence encoding a BLIP protein or a variant thereof, or a nucleotide sequence which is complementary thereto. Preferably the nucleotide sequence is a cDNA sequence. Particularly preferably, the nucleotide sequence is or comprises SEQ ID NO:3.

According to another aspect of the invention there is provided an isolated nucleotide sequence encoding a LIP protein or a variant thereof, or a nucleotide sequence which is complementary thereto. Preferably the nucleotide sequence is a cDNA sequence. Particularly preferably, the nucleotide sequence is or comprises SEQ ID NO:6.

According to another aspect of the invention there is provided an isolated DNA molecule having a sequence selected from the group consisting of: (a) isolated DNA which encodes the BLIP protein of **Figure 2A** (SEQ ID NO: 4); (b) isolated DNA which hybridizes to isolated DNA of (a) above under conditions represented by a wash stringency of 0.3M NaCl, 0.03M sodium citrate, and 0.1% SDS at 60°C, which is at least 65% homologous to the isolated DNA of (a) above, and which encodes a BLIP protein; and (c) isolated DNA having a sequence complementary to (a) - (c), above.

According to another aspect of the invention there is provided an expression vector comprising a nucleic acid sequence as referred to above. According to another aspect of the invention there is provided a nucleic acid construct having a promoter and a heterologous nucleic acid operably linked to said promoter, wherein said heterologous nucleic acid is a nucleic acid molecule as given above

According to another aspect of the invention there is provided a stable cell line comprising a recombinant DNA sequence or an expression vector as referred to above, capable of expressing the encoded a protein. Preferably the cell line is a modified HEK293 or HeLa cell line.

According to another aspect of the invention there is provided a polyclonal or monoclonal antibody that specifically binds to a BLIP protein as given above.

According to another aspect of the invention there is provided an antisense oligonucleotide complementary to a nucleic acid as given above and having a length sufficient to hybridize thereto under physiological conditions; along with DNA encoding such an antisense oligonucleotide; and a nucleic acid construct having a promoter and a heterologous nucleic acid operably linked to said promoter, wherein the heterologous nucleic acid is a DNA encoding such an antisense oligonucleotide.

According to another aspect of the invention there is provided a method of inhibiting NF-κB activation in a cell which comprises providing to the cell an inhibitor of BLIP-related NF-κB activation, in an amount effective to inhibit NF-κB activation therein. The cell may be provided in any suitable form, such as in in vitro culture. The providing step may be carried out by any suitable means, such as by delivering the inhibitory compound into the cell or by delivering into the cell a nucleic acid encoding and expressing an inhibitory protein. The cell is preferably a mammalian cell and more preferably a human cell.

A further aspect of the present invention is the use of a BLIP kinase-dead mutant, or a LIP protein, as an inhibitor of NF-κB activation in a cell, or as an anti-cancer agent or adjuvant.

According to another aspect of the invention there is provided a method for screening compounds for the ability to modulate (enhance or inhibit) BLIP-dependent activation of NF-κB. Such methods comprise contacting a test compound with a BLIP protein as referred to above and detecting modulating activity or inactivity.

According to another aspect of the invention there is provided a compound that modulates BLIP -dependent NF-κB activity, identifiable by the methods referred to above.

According to another aspect of the invention there is provided a method of treatment or prophylaxis of a disorder that is responsive to modulation of BLIP-dependent NF-κB activation, in a mammalian subject, that comprises administering to said patient an effective amount of a compound that modulates (inhibits or enhances) BLIP-dependent NF-κB activation. Preferably the disorder is selected from neoplastic, inflammatory and immune disorders.

According to a further aspect of the invention there is provided use of a compound that modulates of BLIP-dependent NF-κB activation in a method of formulating a medicament for treatment or prophylaxis of a disorder that is responsive to modulation of BLIP-dependent NF-κB activation.

### Brief Description of the Figures

The present invention will be further described by way of example and with reference to the following figures:
**Figure 1** graphs the effect of overexpression of LIP on NF-κB activation; increasing expression of LIP decreased TNFα-induced κB-dependent reporter activity.
**Figure 2A** shows the amino acid sequence of the human BLIP protein (SEQ ID NO:4).
**Figure 2B** is a schematic representation of BLIP showing the FRAP/ATM PI 3-kinase-like domain and the LIP region.
**Figure 3A** is an alignment of the PI 3-kinase domain of human BLIP with that of FRAP. Asterisks indicate amino acids that are identical in both aligned proteins; the conserved critical residues that confer catalytic activity to the protein are boxed.
**Figure 3B** is an alignment of the PI 3-kinase domain of human BLIP with that of ATM. Asterisks indicate amino acids that are identical in both aligned proteins; the conserved critical residues that confer catalytic activity to the protein are boxed.

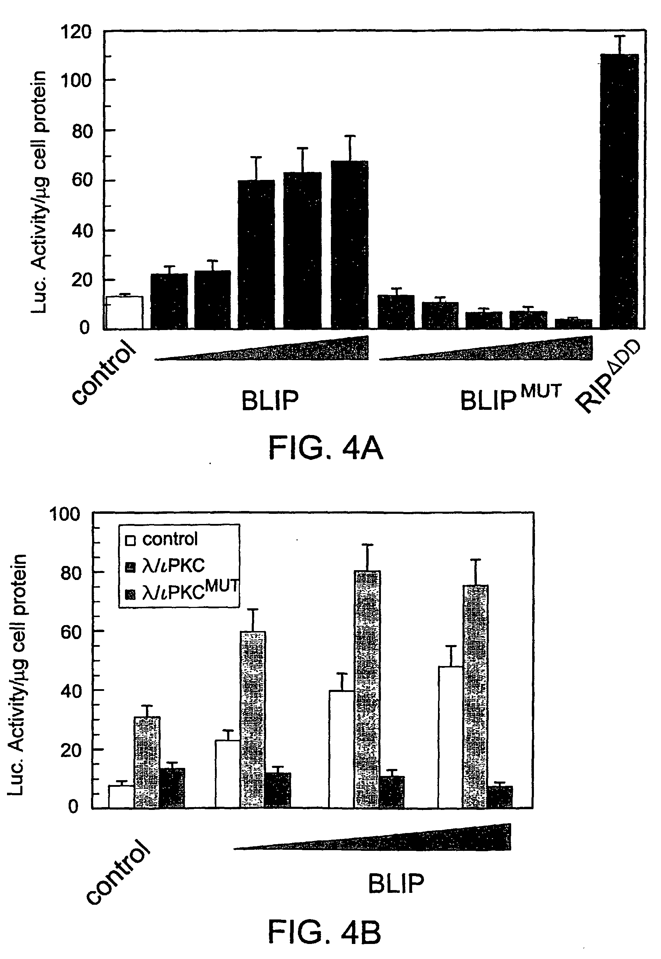

**Figure 4A** graphs the effects of BLIP on NP-κB activation in cultures of human HEK293 cells. The HEK293 cells were transfected with a κB-dependent luciferase reporter plasmid and either: a negative control vector (first bar); increasing concentrations of an expression plasmid for wild-type BLIP (BLIP; second - sixth bars); increasing concentrations of an expression plasmid for kinase-inactive BLIP (BLIP^{MUT:}; seventh - eleventh bars); or an RIP expression vector (positive control; final bar). After 24 hours the levels of luciferase expression were determined. Results are the mean ± SD of three independent experiments with incubations in duplicate.
**Figure 4B** graphs the role of BLIP on NF-κB activation in cultures of HEK293 cells. Cells were transfected with a κB-dependent luciferase reporter plasmid and either: a negative control vector (white bars), an expression plasmid for wild type λ/ 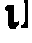 PKC (λ/ 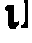 PKC; hatched bars), or an expression plasmid for kinase-inactive λ/ 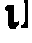 PKC (λ/ 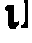 PKC^{MUT}; solid bars). Luciferase activity in cells without BLIP (control) and with increasing concentrations of wild-type BLIP (BLIP) was assessed after 24 hours. Results are the mean ± SD of three independent experiments with incubations in duplicate.
**Figure 5** shows the percentage of HeLa cells with nuclear p65 after transfection with a vector for an HA-tagged version of BLIP^{MUT}. Twenty-hours post-transfection, cells were either untreated (control; white bars) or stimulated for 20 min with TNFα (30 ng/ml; solid bars). Analysis was by immunofluorescence confocal microscopy with monoclonal anti-HA antibody 12CA5 and a goat polyclonal anti-p65 antibody. The graph shows the mean ± SD of several fields (300 cells) from three independent experiments.
**Figure 6** provides the nucleotide sequence (SEQ ID NO:3) of DNA encoding the BLIP protein of SEQ ID NO:4.

### Detailed Description of the Invention

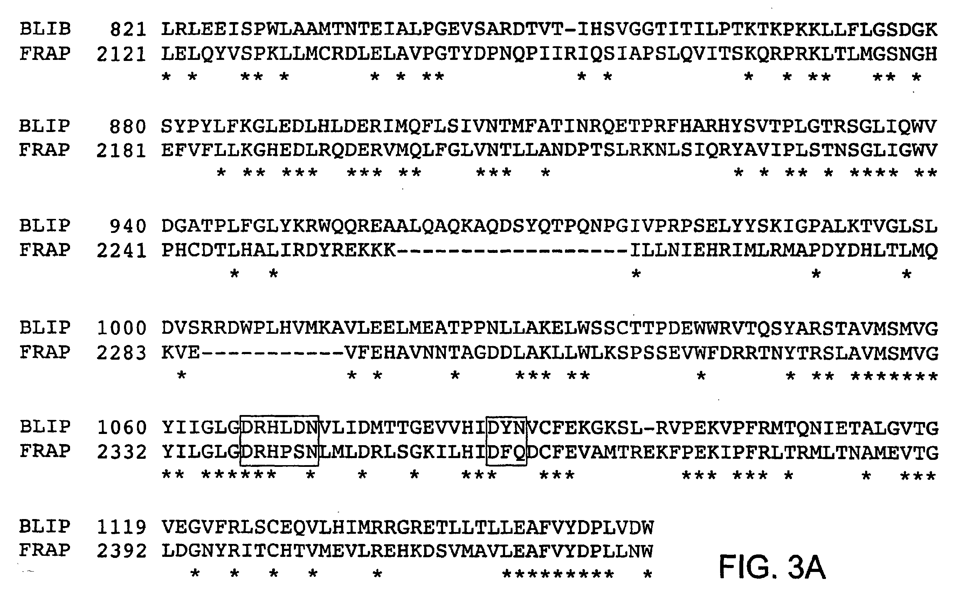

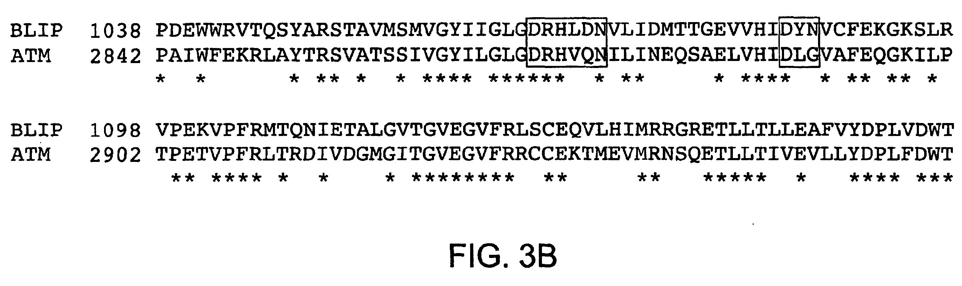

The present inventors have isolated and characterized a novel LIP isoform, herein termed BLIP (for "big LIP") (SEQ ID NO:4). BLIP interacts with λ/ 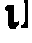 PKC in a TNFα-dependent manner, and activates NF-κB in a λ/ 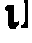 PKC-dependent manner. Of functional relevance, BLIP has a domain that is highly homologous to the PI 3-kinase-like region of FKBP-rapamycin associated protein (FRAP) and Ataxia Telangiectasia Mutated (ATM) protein (see **Figs. 3A** and **3B**). Interestingly, the ectopic expression of wild-type BLIP is sufficient to activate NF-κB in a λ/ 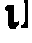 PKC dependent manner, whereas a BLIP kinase-inactive mutant is dominant negative. This is the first evidence of the involvement of a FRAP/ATM-like molecule in the activation of NF-κB. NF-κB has been shown to be relevant in cell proliferation and apoptosis (Baldwin, 1996; Lenardo and Baltimore, 1989). The present inventors further expanded the known sequence of the human LIP protein, and determined that the LIP protein inhibits NF-κB dependent transcription when expressed in a cell.

The atypical PKCs are regulated by protein regulators. Compared to the amino acid sequences of classical or novel PKCs, the sequences of the atypical PKC subfamily differ in the regulatory domain. The identification of selective modulators for each PKC isotype will assist in understanding the function and regulation of the different PKC isoforms. A protein named LIP has been identified as a selective regulator of λ/ 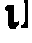 PKC (Diaz-Meco et al., 1996). LIP binds to the zinc finger of λ/ 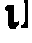 PKC but does not bind to αPKC, εPKC or even to the highly related ζPKC (Diaz-Meco et al., 1996). Interestingly, overexpression of a fragment of LIP (the R4 fragment) that is sufficient to interact with λ/ 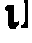 PKC is capable of stimulating a κB-dependent promoter activity (Diaz-Meco et al., 1996).

Previously it was reported that expression of a minimal C-terminal region of LIP (named R4 in Diaz-Meco et al., 1996) that bound λ/ 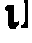 PKC was also sufficient to activate NF-κB (Diaz-Meco et al., 1996). However, the present inventors determined that the overexpression of full-length LIP does not activate NF-κB, and in fact inhibits κB-dependent gene transcription (see Example 4).

LIP was initially identified by the two-hybrid system in yeast. An isolated cDNA of LIP encoding a protein of 713 amino acids with a predicted molecular weight of 79.7 kDa is described by Diaz-Meco et al. (1996). The present inventors determined the sequence of LIP to be 779 amino acids (SEQ ID NO:6), and observed that LIP was expressed in testicle and heart tissues but not in other tissues investigated (liver, ovary, spleen, thymus, brain, lung and kidney). However, a band that reacted with the anti-LIP antibody and having a molecular weight higher than 200 kDa was unexpectedly detected (results not shown).

The expression of LIP and the newly identified band was then determined in two different human cell lines (HEK293 and HeLa cells). See Example 2. It was observed that both bands were detectable in both cell lines although the proportion varied, with the high molecular weight protein (BLIP) more abundant in HEK293 than in HeLa cells (results not shown).

The present inventors assessed whether BLIP could bind to λ/ 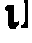 PKC in vivo (Example 3), using HEK293 cells that were either untreated (control) or stimulated with TNFα for various times. Under resting conditions there was little or no association of BLIP or LIP to λ/ 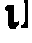 PKC (data not shown). However, upon TNFα stimulation there was a rapid and sustained interaction of BLIP with λ/ 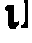 PKC.

To further investigate whether BLIP was physiologically relevant in the aPKC/NF-κB pathway, the present inventors next cloned and molecularly characterized BLIP (Example 5). BLIP is a protein of 2392 amino acids (**Fig. 2A**, SEQ ID NO:4); the region encompassing amino acids 881-1209 of BLIP displays a high degree of homology with the PI 3-kinase domains of FRAP (SEQ ID NO:1) and ATM (SEQ ID NO:2) (**Figs. 3A** and **3B**). The alignment of the PI 3-kinase-like domains of BLIP, FRAP and ATM indicates that BLIP has kinase activity, as the DXXXXN and DFG motifs that constitute critical residues of the catalytic site conserved in all ATM family members with kinase activity, are also conserved in BLIP (see **Figs. 3A** and **3B**).

The present inventors investigated whether the FRAP/ATM-like kinase domain of BLIP plays a role in NF-κB activation (Example 6). HEK293 cells were transfected with a κB-dependent reporter gene (luciferase) along with increasing concentrations of expression plasmids for BLIP, either wild-type BLIP or a kinase-inactive mutant (BLIP^{MUT}). The results (**Figure 4A**) indicate that the expression of wild-type BLIP (but not a kinase inactive mutant BLIP) is sufficient to activate the κB-dependent reporter activity to an extent comparable to that of RIP (Receptor Interacting Protein).

Further experiments established that the NF-κB activation effect of BLIP is dependent on λ/ 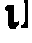 PKC (Example 7). Cells were transfected with a κB-dependent luciferase reporter plasmid and either a negative control vector, an expression plasmid for wild type λ/ 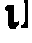 PKC, or an expression plasmid for kinase-inactive λ/ 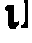 PKC^{*MUT*}. Luciferase activity in cells without BLIP (control) and with increasing concentrations of wild-type BLIP (BLIP) was assessed, and it was seen that the co-expression of wild-type λ/ 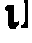 PKC cooperated with BLIP to activate NF-κB, whereas the expression of the kinase-inactive λ/ 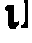 PKC mutant did not. Results are shown in **Figure 4B**.

The present inventors' work indicates that BLIP is a critical component of the NF-κB pathway, and depends on its own kinase activity and that of λ/ 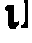 PKC to exert its function.

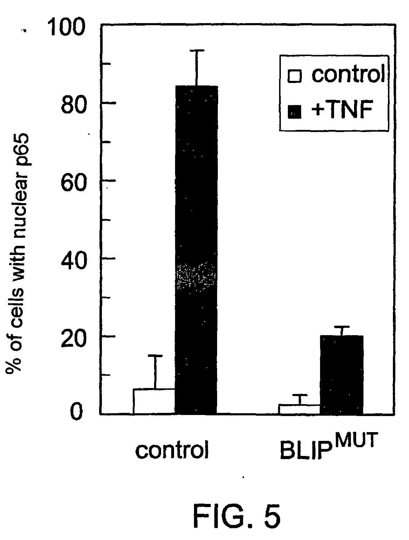

To demonstrate that BLIP controls the translocation of RelA to the nucleus, cells were transfected with the HA-tagged BLIP mutant expression vector (BLIP^{MUT}). Twenty-four hours post-transfection the cells were either stimulated with TNFα or untreated (control). The cells were then analyzed by double immunofluorescence using an anti p65 (RelA) antibody and anti HA-antibody to detect cells that were expressing the construct (data not shown). Analysis of the cells revealed cells transfected with the BLIP mutant had inhibited TNFα-induced translocation of p65 compared to cells transfected with wild-type BLIP **(Fig. 5)**.

The atypical PKCs are involved in the control of NF-κB and p70S6K (Romanelli et al., 1999). Both pathways are critical in the control of inflammatory and immunosuppressor cell function. NF-κB controls a myriad of immune responses and is a potent anti-apoptotic molecule. Inhibition of the atypical PKC kinases blocks the growth and proliferation of cells and initiates a programmed cell death (Berra et al., 1993, 1997; Diaz-Meco et al., 1996; Murray et al., 1997). In addition, the inhibition of aPKCs leads to a reversion of the transforming phenotype of Ras-transformed cells (Bjorkoy et al., 1995), indicating that inhibitors of the atypical PKCs may have anti-neoplastic effects and would be suitable for screening for anti-cancer therapeutic effects. Consistent with this notion, the atypical PKCs target key intermediaries in the cell signaling cascades involved in cell proliferation. It has been demonstrated that ζPKC as well as λ/ 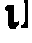 PKC control the activation of the MEK-ERK signaling cascade, along with Raf, in the Ras and the PI 3-kinase γ pathways (Diaz-Meco et al., 1994, 1995; Liao et al., 1997; Schönwasser et al., 1998; Takeda et al., 1999).

The present inventors determined that BLIP has a domain highly homologous to the kinase region of FRAP. FRAP is the target of the immunosupressor molecule rapamycin (Dennis et al., 1999). Rapamycin blocks the activation of p70S6K in T cells which constitutes the molecular basis of its immunosuppressor actions (Dennis et al., 1999). Interestingly, the activity of p70S6K is required and sufficient for the cell cycle progression of T lymphocytes. The BLIP-λ/ 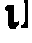 PKC tandem represents an alternate pathway to that of FRAP in the design of potentially novel immunosuppressor molecules.

The results reported herein demonstrate the critical role of BLIP in NF-κB activation, and also demonstrate that the FRAP/ATM-like kinase domain of BLIP is important in NF-κB activation. A kinase-dead mutant of BLIP protein is unable to activate NF-κB and even has a dominant negative activity when transfected in TNFα-activated cells (similar to the effects of the full-length LIP).

While not wishing to be held to a single theory underlying the mechanisms of the LIP and BLIP proteins, the present investigators postulate that the full-length LIP constrains the ability of λ/ 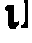 PKC to activate NF-κB. This constraint is not present when the R4 fragment is used to investigate the activation of NF-κB (Diaz-Meco et al., 1996). It is possible that in the case of BLIP, the putative constraint is relieved by the action of the FRAP/ATM-like kinase activity.

Throughout the present specification and the accompanying claims the words "comprise" and "include" and variations such as "comprises", "comprising", "includes" and "including" are to be interpreted inclusively. That is, these words are intended to convey the possible inclusion of other elements or integers not specifically recited, where the context allows.

Amino acid sequences disclosed herein are presented in the amino to carboxy direction, from left to right. The amino and carboxy groups are not presented in the sequence. Nucleotide sequences are presented herein by single strand only, in the 5' to 3' direction, from left to right. Nucleotides and amino acids are represented herein in the manner recommended by the IUPAC-IUB Biochemical Nomenclature Commission, or (for amino acids) by three letter code, in accordance with established usage.

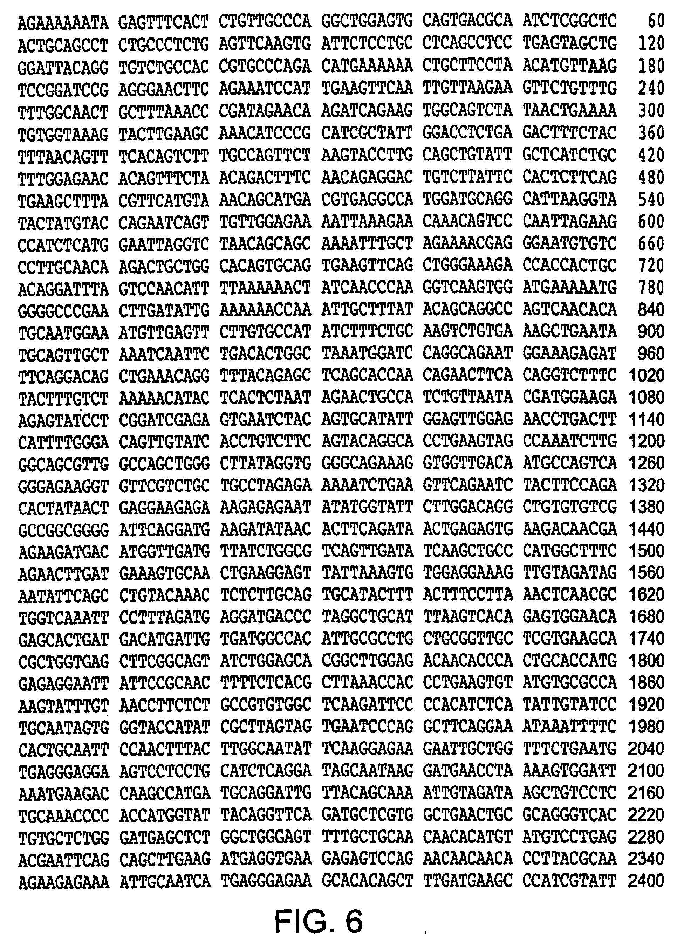

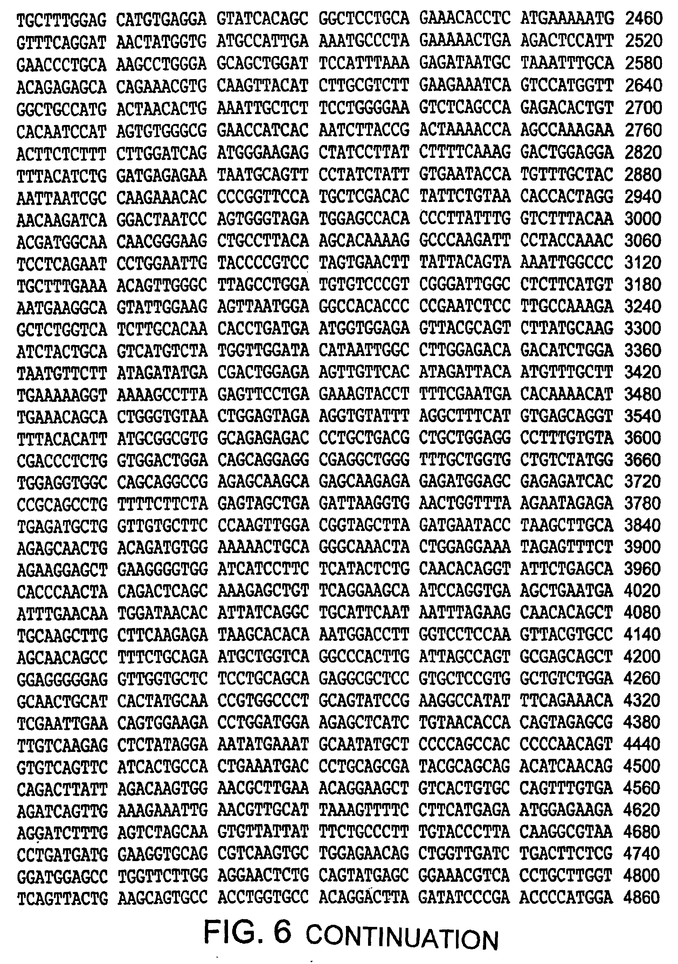

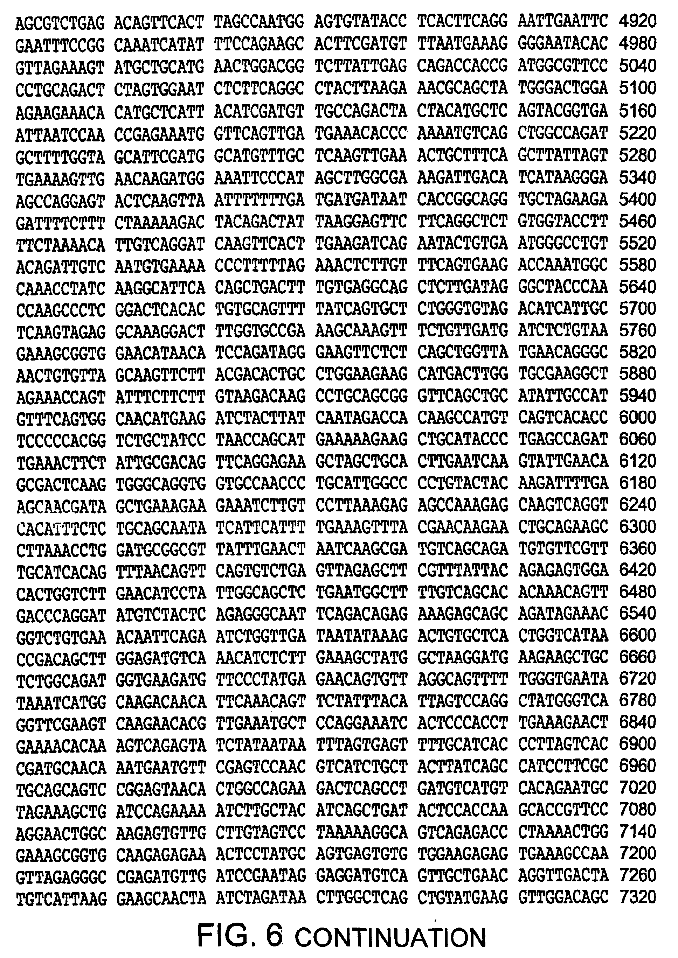

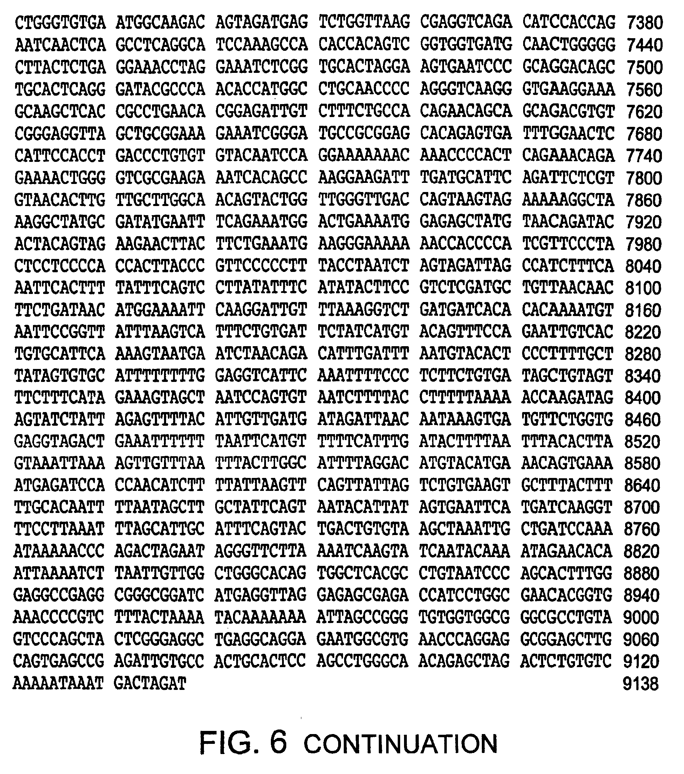

As referred to above, the present invention relates to isolated BLIP and LIP proteins. Sequence information for isolated human BLIP is provided in **Figure 2A** (amino acid; SEQ ID NO:4) and **Fig. 6** (nucleotide; SEQ ID NO:3). Amino acid sequence information for a kinase-inactive mutant of BLIP is provided in SEQ ID NO:5. Sequence information for LIP is provided in SEQ ID NO:6 (amino acid) and SEQ ID NO:7 (nucleotide). In the context of this invention the term "isolated" indicates that the protein or nucleotide molecule is not in its native state, insofar as it has been purified at least to some extent or has been synthetically produced, for example by recombinant methods. The term "isolated" therefore includes the possibility of the molecule being in combination with other biological or non-biological material, such as cells, suspensions of cells or cell fragments, proteins, peptides, expression vectors, organic or inorganic solvents, or other materials where appropriate, but excludes the situation where the protein is in a state as found in nature.

Routine methods known in the art, as further explained in the subsequent experimental section, can be employed to purify and/or synthesise the proteins and nucleotide molecules according to the invention. Such methods are well understood by persons skilled in the art, and include techniques such as those disclosed in Sambrook, J. et al (1), the disclosure of which is included herein in its entirety by way of reference.

The term "BLIP variant" as used throughout the specification and claims refers to other peptides or proteins that retain the same essential functional character of the BLIP protein of SEQ ID NO:4 provided herein, but which vary in amino acid sequence. Variants are also intended to be included within the scope of the invention. For example, peptides or proteins with greater than about 65%, preferably at least 70%, 75%, 80% or 85%, and particularly preferably at least about 90% or even 95% or 98% sequence similarity with SEQ ID NO:4, and which retain BLIP functions, are considered as variants of the BLIP protein. Such variants may include the deletion, modification or addition of single amino acids or groups of amino acids within the protein sequence, as long as the peptide maintains the basic biological functionality of a BLIP protein, i.e., interacts with λ/ 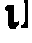 PKC in a TNFα-dependent manner and activates NF-κB in a TNFα-dependent and λ/ 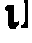 PKC-dependent manner. This biological functionality can be assessed by conducting studies as described herein and as known in the art.

The term "BLIP protein" as used herein does not include a protein consisting only of the LIP protein sequence (SEQ ID NO:6) or the R4 fragment (SEQ ID NO:8).

As used herein, a BLIP protein is one having amino acid SEQ ID NO:4, or having amino acid similarity of at least about 65%, 75%, 80%, 85%, 90%, 95%, 97%, 98% or even 99% to SEQ ID NO:4 and having BLIP functions. A protein having BLIP functions is one that interacts with λ/ 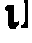 PKC in a TNFα-dependent manner, activates NF-κB in a TNFα-dependent and λ/ 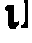 PKC -dependent manner, and contains a functional kinase domain. The degree of amino acid sequence similarity between proteins can be calculated using various commercially available computer software programs (e.g., BLAST analysis, as is known in the art).

As used herein, a kinase inactive mutant of BLIP is a protein or peptide having substantial sequence similarity to a functional BLIP protein (e.g., sequence similarity of at least about 65%, 75%, 80%, 85%, 90%, 95%, 97%, 98% or even 99%), but having a non-functional kinase domain. One skilled in the art will recognize the PI-3 kinase domain of a BLIP protein by its sequence. An example of a kinase-inactive BLIP mutant is provided herein by SEQ ID NO:5.

The term "LIP variant" as used throughout the specification and claims refers to other peptides or proteins that retain the same essential functional character of the LIP protein of SEQ ID NO:6 provided herein, but which vary in amino acid sequence. Variants are also intended to be included within the scope of the invention. For example, peptides or proteins with greater than about 90%, 92%, 95% or even 97% or 98% sequence similarity with SEQ ID NO:6, and which retain LIP functions, are considered as variants of the LIP protein. Such variants may include the deletion, modification or addition of single amino acids or groups of amino acids within the protein sequence, as long as the peptide maintains the basic biological functionality of a LIP protein, i.e., inhibits NF-κB dependent transcription. This biological functionality can be assessed by conducting studies as described herein and as known in the art. As used herein, a LIP protein is one having amino acid SEQ ID NO:6, or having amino acid similarity of at least about 90%, 92%, 95%, 97%, 98% or even 99% to SEQ ID NO:6 and having LIP functions. The degree of amino acid sequence similarity between proteins can be calculated using various commercially available computer software programs (e.g., BLAST analysis, as is known in the art).

In general, those skilled in the art will appreciate that minor deletions or substitutions may be made to the amino acid sequences of peptides and proteins of the present invention without unduly adversely affecting the activity thereof. Thus, proteins containing such deletions or substitutions are a further aspect of the present invention. In peptides containing substitutions or replacements of amino acids, one or more amino acids of a sequence may be replaced by one or more other amino acids wherein such replacement does not affect the function of that sequence. Such changes can be guided by known similarities between amino acids in physical features such as charge density, hydrophobicity/hydrophilicity, size and configuration, so that amino acids are substituted with other amino acids having essentially the same functional properties, as is known in the art.

The invention also includes nucleotide sequences that encode BLIP and LIP proteins, mutants, and variants thereof as well as nucleotide sequences which are complementary thereto. The nucleotide sequence may be RNA or DNA including genomic DNA, synthetic DNA or cDNA. Preferably the nucleotide sequence is a DNA sequence and most preferably, a cDNA sequence. (Nucleotide sequence information is provided in **Figure 6** (SEQ ID NO:3); the BLIP protein of SEQ ID NO:4 is encoded by nucleotides 152 - 7327 of SEQ ID NO:3). Nucleotide molecules can be isolated from human cells or synthesised according to methods known in the art, as described by way of example in Sambrook, J. et al (1), the disclosure of which is included herein in its entirety by reference. The nucleotide molecules according to the invention have utility in production of the proteins according to the invention, which may take place *in vitro, in vivo* or *ex vivo*. The nucleotide molecules may be involed in recombinant protein synthesis or indeed as therapeutic agents in their own right, utilised in gene therapy techniques. Nucleotides complementary to those encoding BLIP proteins, or antisense sequences, may also be used in gene therapy, such as in strategies for down-regulation of expression of the proteins of the invention.

As used herein, DNA molecules that encode a BLIP protein is intended to encompass natural allelic variations in the DNA molecules. Hybridization conditions which permit other DNA sequences which code for a BLIP protein to hybridize to a DNA sequence as given herein (e.g., SEQ ID NO:3) are, in general, high stringency conditions. For example, hybridization of such sequences may be carried out under conditions represented by a wash stringency of 0.3M NaCI, 0.03M sodium citrate, 0.1% SDS at 60°C or even 70°C in a standard in situ hybridization assay. See J. Sambrook et al., Molecular Cloning, A Laboratory Manual (2d Ed. 1989)(Cold Spring Harbor Laboratory)). In general, DNA sequences which code for a BLIP protein and hybridize to a DNA sequence of SEQ ID NO:3 disclosed herein will have at least 65%, 70%, 75%, 80%, 85%, 90%, 92% or even 95% nucleotide sequence similarity with SEQ ID NO:3, and encode a protein have BLIP functions. Sequence similarity may be determined using commercially available computer software programs (e.g., BLAST analysis as is known in the art). Isolated DNA of the present invention can be of any species of origin, including mouse, rat, rabbit, cat, porcine, and human, but is preferably of mammalian origin and most preferably of human origin.

As used herein, a DNA molecule encoding a LIP protein is intended to encompass natural allelic variations in the DNA molecules. Hybridization conditions which permit other DNA sequences which code for a LIP protein to hybridize to a DNA sequence as given herein (e.g., SEQ ID NO:7) are, in general, high stringency conditions. For example, hybridization of such sequences may be carried out under conditions represented by a wash stringency of 0.3M NaCl, 0.03M sodium citrate, 0.1% SDS at 60°C or even 70°C in a standard in situ hybridization assay. See J. Sambrook et al., Molecular Cloning, A Laboratory Manual (2d Ed. 1989)(Cold Spring Harbor Laboratory)). In general, DNA sequences which code for a LIP protein and hybridize to a DNA sequence of SEQ ID NO:3 disclosed herein will have at least 65%, 70%, 75%, 80%, 85%, 90%, 92% or even 95% nucleotide sequence similarity with SEQ ID NO:3, and encode a protein have LIP functions. Sequence similarity may be determined using commercially available computer software programs (e.g., BLAST analysis as is known in the art). Isolated DNA of the present invention can be of any species of origin, including mouse, rat, rabbit, cat, porcine, and human, but is preferably of mammalian origin and most preferably of human origin.

Further, oligonucleotide molecules that code for the BLIP or LIP proteins (or mutants or variants thereof), but which differ in codon sequence from the foregoing DNA molecules due to the degeneracy of the genetic code, are also an aspect of this invention. The degeneracy of the genetic code, which allows different nucleic acid sequences to code for the same protein or peptide, is well known in the art. Oligonucleotide molecules that code for the same BLIP protein as coded for by the foregoing sequences, but which differ in codon sequence from these due to site directed mutigenesis are yet another aspect of this invention. Site directed mutagenesis techniques are known in the art.

The present invention also includes expression vectors that comprise nucleotide sequences encoding the present proteins, mutants or variants thereof. Such expression vectors are routinely constructed in the art of molecular biology and may for example involve the use of plasmid DNA and appropriate initiators, promoters, enhancers and other elements, such as for example polyadenylation signals which may be necessary, and which are positioned in the correct orientation to allow protein expression. Suitable vectors will be apparent to persons skilled in the art. By way of further example in this regard we refer to Sambrook et al (1), the disclosure of which is included herein in its entirety.

Vectors may be used either to amplify DNA encoding a desired protein and/or to express DNA which encodes a desired protein. The DNA sequence encoding the desired protein is operably linked to suitable control sequences capable of effecting the expression of the protein in a suitable host. DNA regions are operably linked or operably associated when they are functionally related to each other. For example, a promoter is operably linked to a coding sequence if it controls the transcription of the sequence; or a ribosome binding site is operably linked to a coding sequence if it is positioned so as to permit translation. The need for such control sequences will vary depending upon the host selected and the transformation method chosen. Vectors may include plasmids, viruses (e.g., adenovirus, cytomegalovirus), phage, and integratable DNA fragments (i.e., fragments integratable into the host genome by recombination).

The invention also includes cell lines that have been modified or transfected to express the proteins of the present invention. Such cell lines include transient, or preferably stable higher eukaryotic cell lines (such as mammalian cells or insect cells), lower eukaryotic cells (such as yeast) or prokaryotic cells (such as bacterial cells). It is also possible for the proteins of the invention to be transiently expressed in a cell line, such as for example in a baculovirus expression system.

According to another aspect, the present invention also relates to antibodies (polyclonal or preferably monoclonal antibodies) that specifically bind to the proteins of the present invention (i.e., antibodies which bind to a single antigenic site or epitope on the proteins). Such antibodies are preferably of monoclonal origin. The antibodies are preferably IgG antibodies of any suitable species, such as rat, rabbit, or horse, but are generally of mammalian origin. Fragments of IgG antibodies which retain the ability to specifically bind the proteins of the present invention, such as F(ab')₂, F(ab'), and Fab fragments, are intended to be encompassed by the term "antibody" herein. See generally E. Harlow and D. Lane, Antibodies: A Laboratory Manual (1988)(New York: Cold Spring Harbor Laboratory Press). The antibodies may be chimeric, as described by M. Walker et al., Molecular Immunol. 26, 403 (1989). Antibodies that bind to BLIP proteins, but not to shorter LIP proteins, are particularly preferred.

Such antibodies are useful in purification, isolation or screening involving immunoprecipitation techniques and may be used as tools to further elucidate the BLIP protein function, or as therapeutic agents in their own right.

Nucleic acid oligodeoxynucleotides compementary to and capable of hybridizing to the sense strand of a gene, or to an mRNA transcribed from that gene, are antisense oligodeoxynucleotides. When the sense strand of a target gene, or an mRNA, is exposed to its antisense oligodeoxynucleotides, interaction of the two will occur such that the gene will be blocked from transcription or the mRNA blocked from translation. The present invention includes oligomers capable of hybridizing to a portion of a gene (or an mRNA) encoding the BLIP or LIP protein. When such an oligomer is hybridized to the target molecule, production of the protein will be substantially prevented or reduced (compared to that which would occur in the absence of the antisense oligonucleotide). Substantial prevention means that the protein is not produced, or is produced at non-functional levels.

Antisense oligonucleotides (and nucleic acids that express such antisense molecules) may be made in accordance with conventional techniques. See, e.g., U.S. Pat. No. 5,023,243 to Tullis; U.S. Pat. No. 5,149,797 to Pederson et al. The length of the antisense oligonucleotide (i.e., the number of nucleotides therein) is not critical so long as it binds selectively to the intended location; this can be determined in accordance with routine procedures. In general, the antisense oligonucleotide will be from 8, 10 or 12 nucleotides in length up to 20, 30, or 50 nucleotides in length. Such antisense oligonucleotides may be oligonucleotides wherein at least one, or all, of the intemucleotide bridging phosphate residues are modified phosphates, such as methyl phosphonates, methyl phosphonothioates, phosphoromorpholidates, phosphoropiperazidates and phosphoramidates. For example, every other one of the internucleotide bridging phosphate residues may be modified as described. In another nonlimiting example, such antisense oligonucleotides are oligonucleotides wherein at least one, or all, of the nucleotides contain a 2' loweralkyl moiety (e.g., C1-C4, linear or branched, saturated or unsaturated alkyl, such as methyl, ethyl, ethenyl, propyl, 1-propenyl, 2-propenyl, and isopropyl). For example, every other one of the nucleotides may be modified as described. See also P. Furdon et al., Nucleic Acids Res. 17, 9193-9204 (1989); S. Agrawal et al., Proc. Natl. Acad. Sci. USA 87, 1401-1405 (1990); C. Baker et al., Nucleic Acids Res. 18, 3537-3543 (1990); B. Sproat et al., Nucleic Acids Res. 17, 3373-3386 (1989); R. Walder and J. Walder, Proc. Natl. Acad. Sci. USA 85, 5011-5015 (1988).

A further aspect of the present invention is screening methods designed to identify compounds that modulate BLIP activation of λ/ 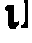 PKC, or that modulate NF-κB activation. By the term "modulation" it is meant that there will be either an increase or a decrease in NF-κB activation in the presence of the compound (compared to that which would be observed in the absence of the compound); such activity generally will result from ligand binding of the compound to BLIP, λ/ 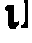 PKC, the tandem of BLIP and λ/ 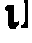 PKC, or to NF-κB itself.

In general terms, such screening methods involves administering a test compound to a cell and detecting BLIP-related activation of NF-κB, compared to a control cell in which the test compound (or functional BLIP protein) is absent. Activation of NF-κB may be assessed using a κB-dependent reporter gene (such as luciferase), or by measuring translocation of NF-κB to the cell nucleus (using methods that are known in the art, e.g., the use of anti-Rel65 antibody to detect nuclear NF-κB). Such screening methods may further comprise detecting whether said test compound binds to λ/ 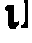 PKC (or BLIP, or NF-κB), e.g., by preparing cell extracts and immunoprecipitating the protein of interest, and immunoblotting using an antibody directed to the test compound.

The ability of compounds to modulate BLIP autophosphorylation activity can also be used as a method of screening compounds for NF-κB activation or inhibitory effects. In general terms, such screening methods involves administering a test compound to a cell and detecting BLIP autophosphorylation, compared to a control cell in which the test compound is absent.

The present invention also includes within its scope those compounds that are identified as possessing useful NF-κB modulation activity, by the screening methods referred to above.

Another aspect of the present invention is the use of compounds that inhibit or enhance NF-κB activation (including compounds identified by the screening techniques referred to above), in the treatment or prophylaxis of disorders which are responsive to modulation of BLIP activity or modulation of NF-κB activity, in a mammalian (preferably human) patient. The present invention thus provides a method of inhibiting (or enhacing) NF-κB activation in a cell, by providing to the cell a compound that inhibits (or enhances) BLIP-dependent NF-κB activation. An example of such an inhibitory compound is a kinase dead BLIP mutant (e.g., a protein of SEQ ID NO:5), or an antisense nucleotide as described above, where the inhibitory compound is given in an amount effective to decrease endogenous BLIP-dependent NF-κB activation in the cell (compared to NF-κB activation that would occur in the cell in the absence of the inhibitory compound). Compounds that enhance NF-κB activation are provided in an amount effective to increase endogenous BLIP-dependent NF-κB activation in the cell (compared to NF-κB activation that would occur in the cell in the absence of the compound). The providing step may be carried out by any suitable means, such as by delivering the inhibitory compound into the cell, or by delivering into the cell a nucleic acid encoding and expressing the inhibitory compound.

Compounds that inhibit the BLIP-related activation of NF-κB will have therapeutic benefit in pathologies where such activation is upregulated. In many cells of the immune system NF-κB is activated in response to various pathogenic signals, and induces transcription of a variety of genes encoding immunologically relevant proteins. Baeuerle and Henkel, *Annu Rev. Immunol*. 12:141 (1994). Interference with the activation or activity of NF-κB may suppress immunological reactions, including that seen in toxic/septic shock, graft-vs-host reactions, acute inflammatory reactions and radiation damage. In cancerous or neoplastic cells, both chemotherapy and radiation therapy can activate NF-κB, which in turn reduces the apoptotic effects of the therapy. Inhibition of NF-κB activation in tumors has been reported to enhance the apoptotic potential of cancer treatments. Wang et al., *Nature Medicine* 5:412 (1999).

Accordingly, BLIP-mediated NF-κB activation is implicated in immunological and neoplastic disease. Therefore, inhibition of BLIP-mediated NF-κB activation will provide a positive therapeutic benefit where immune suppression is desired, or where anti-neoplastic effects are desired. In particular, such inhibitory compounds will have utility for treatment and/or prophylaxis of disorders such as toxic/septic shock, graft-vs-host reactions, acute inflammatory reactions and radiation damage. Such compounds will further have use as anti-neoplastic agents in the treatment of tumors or cancers, and as adjuvants in the chemotherapy or radiation therapy of neoplastic growths. For example, adenoviral delivery to cancer cells of a kinase-dead mutant of BLIP (or another inhibitor of BLIP-dependent NF-κB activation), will be useful as an adjuvant in the chemotherapy of cancers, to increase the apoptotic effects of the chemotherapies.

Compounds that increase or enhance the BLIP-related activation of NF-κB will have therapeutic benefit in pathologies where such activation is reduced, or where it is desired to upregulate NF-κB activation. Such compounds are useful as immunostimulators and as inhibitors of cell death.

NF-κB modulating compounds, including those identified according to the screening methods outlined above, may be formulated with standard pharmaceutically acceptable carriers and/or excipients as is routine in the pharmaceutical art, and as fully described in Remmington's Pharmaceutical Sciences, Mack Publishing Company, Eastern Pennsylvania, 17th Ed, 1985, the disclosure of which is included herein in its entirety by way of reference. The compounds may be administered via enteral or parenteral routes such as via oral, buccal, anal, pulmonary, intravenous, intraarterial, intramuscular, intraperitoneal, topical or other appropriate administration routes.

The present invention will now be further described, by way of example, in the appended experimental section.

### Example 1

### Materials and Methods

### Reagents and Cell Cultures:

Recombinant human TNFα was purchased from Promega; monoclonal 12CA5 anti-Hemagglutinin (anti-HA) antibody from Boehringer; monoclonal antibody against λPKC from Transduction Laboratories; goat polyclonal anti-p65 (NF-κB) antibody from Santa Cruz Biotechnologies, Inc. The rabbit affinity-purified anti-LIP has been described (Díaz-Meco et al., 1996). Human HEK293 and HeLa cells were obtained from the American Type Culture Collection (ATCC). Cultures of HEK293 cells were maintained in high glucose Dulbecco's modified Eagle's medium containing 10% fetal calf serum, penicillin G (100 µg/ml), and streptomycin (100 µg/ml) (Flow). HeLa cells were maintained in minimum essential medium Eagle supplemented with 0.1 mM non-essential amino acids, 1.0 mM sodium piruvate and 10% fetal calf serum. Subconfluent cells were transfected by the calcium phosphate method (Clontech Laboratories, Inc.).

### Plasmids:

To clone the cDNA for BLIP coding sequence, a human brain Marathon Ready cDNA (Clontech Laboratories, Inc.) was used following the manufacturer's procedures. This cDNA was subcloned into the NotI site of the pCDNA3-HA vector to make the pCDNA3-HA-BLIP construct. pCDNA3-HA-BLIP^{MUT} (D1066A; N1071K) was obtained by site directed mutagenesis (Quick Change, Stratagene). pCDNA3-HA-λ/ 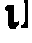 PKC and pCDNA3-HA-λ/ 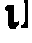 PKC^{MUT} have previously been described (Díaz-Meco et al., 1996).

### Immunoprecipitations:

For immunoprecipitations of endogenous proteins, HEKI293 cells were used. Some cells were stimulated with 30 ng/ml of TNF-α. Cells were then harvested and lysed in buffer PD [40 mM Tris-HCl (pH 8.0), 500 mM NaCl, 0.1% Nonidet P-40, 6 mM EDTA, 6 mM EGTA, 10 mM β-glycerophosphate, 10 mM NaF, 10 mM PNPP, 300 µM Na₃VO₄, 1mM benzamidine, 2 M PMSF, aprotinin 10 µg/ml, leupeptin 1µg/ml, pepstatin 1 µg/ml, 1mM DTT], and lmg of whole-cell lysate was diluted in PD buffer and incubated with 10 µg of the monoclonal anti-λ/ 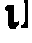 PKC antibody. This reaction mixture was incubated on ice for 2 hours, and then 25 µl of protein A or G beads was added and the mixture was left to incubate with gentle rotation for an additional 1 hour at 4°C. The immunoprecipates were then washed three times with PD buffer. Samples were fractionated on 8% SDS-PAGE, transferred to Nitrocellulose ECL membrane (Amersham) and subjected to Western blot analysis with anti-LIP. Proteins were detected with the ECL reagent (Amersham).

### Luciferase reporter assays:

Subconfluent cultures of HEK293 cells were transfected with different amounts of plasmid pCDNA3-HA-BLIP or pCDNA3-HA-BLIP^{MUT} together with the κB-Luc reporter plasmid either with or without expression vectors for wild type and dominant negative λ/ 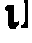 PKC(pCDNA3-HA-λ/ 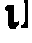 PKC and pCDNA3-HA-λ/ 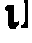 PKC^{MUT}). After 24 hours, cells were stimulated with TNF-α for 4 hours. Extracts were prepared and luciferase activity determined as described (Díaz-Meco et al., 1996).

### Immunofluorescence:

HeLa cells were grown on glass coverslips in growth media. Subconfluent cells were transfected with 5 µg of HA-BLIP^{MUT}. Twenty-hours post-transfection, cells were stimulated with TNF-α (30 ng/ml) for 20 min. Cells were rapidly washed twice in ice-cold PBS, and fixed in 4% formaldehyde for 15 minutes at room temperature. Cells were washed four times with PBS and permeabilized with 0.1 % Triton X-100. Free aldehyde groups were quenched with 50 mM NH4Cl. Endogenous peroxidase activity was quenched by treatment with 1% H₂O₂ in PBS for 15 minutes. The fixed cells were blocked in blocking solution. Cells were incubated with the different antibodies for 1 hour at 37°C. Transfected HA-tagged protein was visualized with the monoclonal 12CA5 anti-HA (Boehringer Mannheim), and an FITC-conjugated anti-mouse (Cappel), and the endogenous p65 with a goat polyclonal antibody (Santa Cruz Biotech), and the tetramethylrhodamine tyramide TSA-Direct amplification system (NEN Life Science Products). Glass coverslips were mounted on Mowiol and were examined with an MRC 1024 Bio-Rad confocal system (Bio-Rad Richmond, CA) mounted on a Zeiss Axiovert 135 microscope (Zeiss, Oberkochen, Germany).

### Example 2

### Detection of BLIP

In the course of a series of immunoblot analysis to determine the expression of LIP in different murine organs and tissues, the present inventors observed that LIP was expressed in testicle and heart tissues but not in other tissues investigated (liver, ovary, spleen, thymus, brain, lung and kidney). However, a band that reacted with the anti-LIP antibody and having a molecular weight higher than 200 kDa was unexpectedly detected (results not shown).

The expression of LIP and the newly identified band was then determined in two different human cell lines routinely used in the laboratory (HEK293 cells and HeLa cells). It was observed that both bands were detectable in both cell lines although the proportion varied, with the high molecular weight protein more abundant in HEK293 than in HeLa cells (results not shown).

**Example 3**

**Binding of BLIP to λ/** 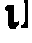 **PKC**

The present inventors assessed whether BLIP could bind to λ/ 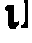 PKC in vivo. Using HEK293 cells, the cells were either untreated (control) or stimulated with TNFα for various times, after which cell extracts were prepared, immunoprecipitated with a selective anti-λ/ 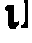 PKC antibody, and analyzed by immunoblotting with the anti-LIP antibody (results not shown). Under resting conditions there was little or no association of BLIP (or LIP; also not shown) to λ/ 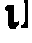 PKC. However, upon TNFα stimulation there was a rapid and sustained interaction of BLIP with λ/ 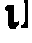 PKC. This indicates that BLIP displays a stimulus-induced association with λ/ 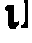 PKC.

### Example 4

### LIP Inhibits NF-κB Dependent Transcription

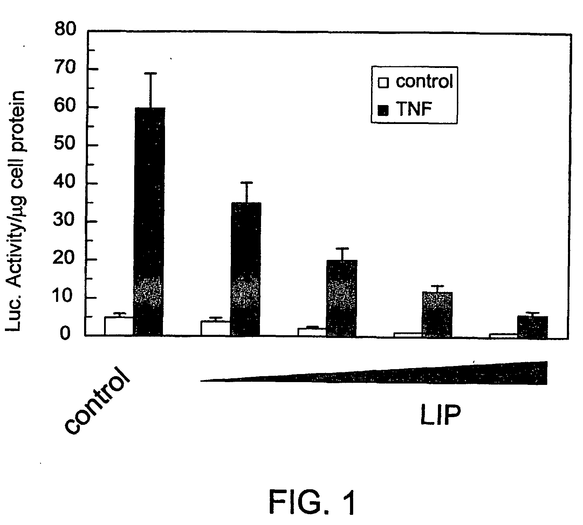

To determine the effect of over-expressing LIP (SEQ ID NO:6) on NF-κB activation, HEK293 cells were transfected with a κB-dependent reporter gene (luciferase) along with increasing concentrations of an expression plasmid for LIP (0.2, 0.5, 1.0 and 2.0 µg/60mm diameter well). Twenty-hour post-transfection cells were either untreated or stimulated with 30 ng/ml TNFα. The results (mean ± SD of three independent experiments with incubation in duplicate; **Figure 1**) demonstrate that the expression of LIP decreases TNFα-induced κB-dependent reporter activity.

### Example 5

### Cloning of BLIP

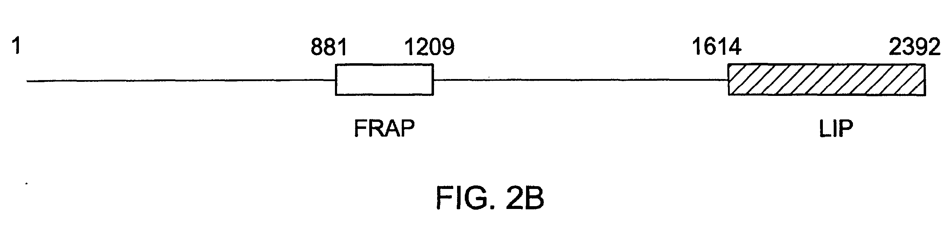

The present inventors next cloned and molecularly characterized human BLIP. The BLIP clone (SEQ ID NO:3) expanded the LIP sequence previously reported by Diaz-Meco et al. (1996). BLIP was found to be a protein of 2392 amino acids **(Fig. 2A,** SEQ ID NO:4) with a predicted molecular weight of 269 kDa. The region encompassing amino acids 881-1209 of BLIP displays a high degree of homology with the PI 3-kinase domains of FRAP and ATM (**Figs. 3A** and **3B**). The alignment of the PI 3-kinase-like domains of BLIP, FRAP and ATM indicates that BLIP has kinase activity, as the DXXXXN and DFG motifs that constitute critical residues of the catalytic site conserved in all ATM family members with kinase activity, are also conserved in BLIP. In contrast to FRAP and ATM, however, the kinase region of BLIP is not located at the most C-terminal part of the molecule but roughly in the middle (**Fig. 2B**). The C-terminus portion of BLIP is the sequence corresponding to LIP. Therefore, LIP constitutes a domain of BLIP (**Fig. 2B**), revealing a domain organization that differs from other PI 3-kinases. Upstream of the PI 3-kinase domain of FRAP there are a series of protein-protein interaction modules called the HEAT domains (Dennis et al., 1999). Such domains (or other regions through which one could predict interactions with other proteins) are absent in the BLIP molecule, with the exception of the LIP region whereby λ/ 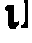 PKC interacts (Dennis et al., 1999). Another feature of FRAP that is absent in LIP is the FRB domain; this domain is the region responsible for the interaction of FRAP with the FKB/rapamycin complex (Dennis et al., 1999). This suggests that BLIP is rapamycin-insensitive.

### Example 6

### Kinase Domain of BLIP

To investigate whether the FRAP/ATM-like kinase domain of BLIP plays a role in NF-κB activation, HEK293 cells were transfected with a κB-dependent reporter gene along with increasing concentrations (0.2, 0.5, 1.0, 2.0 µg/60mm diameter well) of expression plasmids for BLIP (either wild-type (SEQ ID NO:4) or a kinase-inactive mutant (SEQ ID NO:5)). As a positive control, cells were also transfected with a RIP (Receptor Interacting Protein) expression vector (RIP is a potent activator of NF-κB). The results (**Figure 4A**) indicate that the expression of wild-type BLIP (but not mutant BLIP) is sufficient to activate the κB-dependent reporter activity to an extent comparable to that of RIP.

**Example 7**

**λ/** 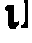 **PKC and BLIP-related NF-κB activation**

To determine whether the NF-κB activation effect of BLIP is dependent on λ/ 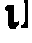 PKC, HEK293 cells were transfected with a κB-dependent luciferase reporter plasmid and one of the following: a negative control vector, an expression plasmid for wild type λ/ 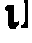 PKC, or an expression plasmid for kinase-inactive λ/ 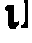 PKC. Luciferase activity in cells without BLIP (control) and with increasing concentrations (0.5, 1.0, 2.0 µg/60mm diameter) of wild-type BLIP (BLIP) was assessed after 24 hours. Results are shown in **Figure 4B**. The co-expression of wild-type λ/ 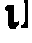 PKC cooperates with BLIP to activate NF-κB, whereas the expression of the kinase-inactive λ/ 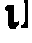 PKC mutant abrogates this effect of BLIP.

### Example 8

### Translocation of RelA to the Cell Nucleus

To further demonstrate that BLIP controls the actual translocation of RelA to the nucleus, HeLa cells were transfected with the HA-tagged BLIP mutant expression vector (BLIP^{MUT}); as control cells, non-transfected HeLa cells were used. Twenty-four hours post-transfection the cells were either stimulated with TNFα for 20 minutes or untreated (control). The cells were then analyzed by double immunofluorescence using an anti p65 (RelA) antibody and anti HA-antibody to detect cells that were expressing the construct (data not shown). Analysis of the cells indicates that more than of the 80% of cells transfected with wildtype BLIP mutant had TNFα-induced translocation of p65, compared to about 20% of cells transfected with BLIP mutant (**Figure 5**).

### Example 9

### Screening Methods

Using an appropriate mammalian cell line (such as HEK293 or HeLa cells), a test compound is administered to the test cells; no test compound is administered to control cells. The cells are then stimulated with TNFα for various times, after which cell extracts are prepared, immunoprecipitated with a selective anti-λ/ 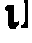 PKC antibody, and analyzed by immunoblotting with the anti-BLIP antibody. An increase in the association of BLIP with λ/ 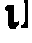 PKC in the test cells (compared to the association found in control cells) indicates that the test compound enhances BLIP-dependent λ/ 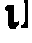 PKC activation (and thus NF-κB activation); a decrease indicates the test compound inhibits BLIP-dependent λ/ 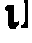 PKC activation (and thus NF-κB activation).

Alternatively, the cells may be transfected with a κB-dependent reporter gene, such as luciferase, and the activity of the reporter gene assessed as a measure of NF-κB activation in the presence of a test compound, compared to control cells.

### References

1. Sambrook, J., Fritsch, E.F. and Maniatis, T. Molecular Cloning: a Laboratory Manual. 2^{nd} Edition. CSH Laboratory Press. (1989)
2. Akimoto, K., K. Mizuno, S. Osada, S. Hirai, S. Tanuma, K. Suzuki, and S. Ohno. 1994. A new member of the third class in the Protein Kinase C Family, PKC, expressed dominantly in an undifferentiated mouse embryonal carcinoma cell line and also in many tissues and cells. J. Biol. Chem. 269: 12677-12683.
3. Baeuerle, P.A., and T. Henkel. 1994. Function and activation of NF-κB in the immune system. Annu. Rev. Immunol. 12: 141-179.
4. Baldwin, A.S. 1996. The NF-κB and IκB proteins: new discoveries and insights. Annu. Rev. Immunol. 14: 649-683.
5. Berghe, W.V., S. Plaisance, E. Boone, K. De Bosscher, M.L. Schmitz, W. Fiers, and G. Haegeman. 1998. p38 and the extracellular signal-regulated kinase mitogen-activated protein kinase pathways are required for nuclear factor-κB p65 transactivation mediated by tumor necrosis factor. J. Biol. Chem. 273: 3285-3290.
6. Berra, E., M.T. Diaz-Meco, J. Lozano, S. Frutos, M.M. Municio, P. Sanchez, L. Sanz, and J. Moscat. 1995. Evidence for a role of MEK and MAPK during signal transduction by protein kinase C ζ. EMBO J. 14: 6157-6163.
7. Bjorkoy, G., A. Overvatn, M.T. Díaz-Meco, J. Moscat, and T. Johansen. 1995. Evidence for a bifurcation of the mitogenic signaling pathway activated by ras and phosphatidylcholine-hydrolyzing. J. Biol. Chem. 270:21299-21306.
8. Dennis, PB., S. Fumagalli, and G. Thomas. 1999. Target of rapamacyn (TOR): balancing the opposite forces of protein synthesis and degradation. Curr. Opin. Gen. & Devel. 9: 49-54.
9. Diaz-Meco, M.T., E. Berra, M.M. Municio, L. Sanz, J. Lozano, V. Díaz-Golpe, M.T. Lain de Lera, J. Alcamí, C.V. Payá, F. Arenzana-Seisdedos, J.L. Virelizier, and J. Moscat. 1993. A dominant negative protein kinase C ζ subspecies blocks NF-κB activation. Mol.Cell.Biol. 13: 4770-4775.
10. Díaz-Meco, M.T., J. Lozano, M.M. Municio, E. Berra, S. Frutos, L. Sanz, and J. Moscat. 1994. ζPKC Induces Phosphorylation and Inactivation of IκB-α In Vitro. EMBO J. 13: 2842-2848.
11. Diaz-Meco, M.T., J. Lozano, M.M. Municio, E. Berra, S. Frutos, L. Sanz, and J. Moscat. 1994. Evidence for the in vitro and in vivo interaction of Ras with protein kinase C ζ. J. Biol. Chem. 269: 31706-31710.
12. Diaz-Meco, M.T., M.M. Municio, P. Sanchez, J. Lozano, and J. Moscat. 1996. Lambda-interacting protein, a novel protein that specifically interacts with the zinc finger domain of the atypical protein kinase C isotype λ/ 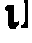 and stimulates its kinase activity *in vitro* and *in vivo.* Mol. Cell. Biol. 16:105-114.
13. DiDonato, J.A., M. Hayakawa, D.M. Rothwarf, E. Zandi, and M. Karin. 1997. A cytokine-responsive IκB kinase that activates the transcription factor NF-κB. Nature 388: 548-554.
14. Dominguez, I., L. Sanz, F. Arenzana-Seisdedos, M.T. Díaz-Meco, J.L. Virelizier, and J. Moscat. 1993. Inhibition of protein kinase ζ subspecies blocks the activation of a NF-κB-like activity in *Xenopus laevis* oocytes. Mol.Cell.Biol. 13:1290-1295.
15. Folgueira, L., J.A. McElhinny, G.D. Bren, W.S. MacMorran, M.T. Díaz-Meco, J. Moscat, and C.V. Payá. 1996. Protein kinase C-ζ mediates NF-κB activation in human immunodeficiency virus-infected monocytes. J. Virol. 70: 223-231.
16. Jung, M., Y. Zhang, S. Lee and A. Dritschilo. 1995. Correction of radiation sensitivity in ataxia telangiectasia cells by a truncated I kappa B-alpha. Science 268:1619-21.
17. Lallena, M. J., M.T. Diaz-Meco, G. Bren, C.V. Payá, and J. Moscat. 1999. Activation of IκB Kinase b by Protein Kinase C Isoforms. Mol. Cell. Biol. 19: 2180-2188.
18. Lenardo, M., and D. Baltimore. 1989. NF-κB: a pleitropic mediator of inducible and tissue-specific gene control. Cell 58: 227-229.
19. Liao, D.F., B. Monia, N. Dean, and B.C. Berk. 1997. Protein kinase C-ζ mediates angiotensin II activation of ERK1/2 in vascular smooth muscle cells. J. Biol. Chem. 272: 6146-6150.
20. Lozano, J., E. Berra, M.M. Municio, M.T. Diaz-Meco, I.Dominguez, L. Sanz, and J. Moscat. 1994. Protein kinase C ζ isoform is critical for κB-dependent promoter activation by sphingomyelinase. J. Biol. Chem. 269:19200-19202.
21. Malinin, N.L., M.P. Boldin, A.V. Kovalenko, and D. Wallach. 1997. MAP3K-related kinase involved in NF-κB induction by TNF, CD95 and IL-1. Nature 385: 540-544.
22. Mercurio, F., H. Zhu, B.W. Murray, A. Shevchenko, B.L. Bennett, J.W. Li, D.B. Young, M. Barbosa, M. Mann, A. Manning, and A. Rao. 1997. IKK-1 and IKK-2: cytokine-activated IκB kinases essential for NF-κB activation. Science 278: 860-866.
23. Müller, G., M. Ayoub, P. Storz, J. Rennecke, D. Fabbro, and K. Pfizenmaier. 1995. PKCζ is a molecular switch in signal transduction of TNFα, bifunctionally regulated by ceramide and arachidonic acid. EMBO J. 14: 1961-1969.
24. Murray, N.R. and A.P. Fields. 1997. Atypical protein kinase C iota protects human leukemia cells against drug-induced apoptosis. J. Biol. Chem. 272: 27521-27524.
25. Nakanishi, H., K.A. Brewer, and J.H. Exton. (1993). Activation of the isozyme of protein kinase C by phosphatidylinositol 3,4,5-trisphosphate J. Biol. Chem. 268: 13-16.
26. Réginer, C.H., H.Y. Song, X. Gao, D.V. Goeddel, Z. Cao, and M. Rothe. 1997. Identification and characterization of an IκB kinase. Cell 90: 373-383.
27. Romanelli, A. K. A. Martin, A. Toker, and J. Blenis. 1999. P70 S6 kinase is regulated by protein kinase c zeta and participates in a phosphoinositide 3-kinase-regulated signalling complex. Mol. Cell Biol. 19:2921-8.
28. Rzymkiewicz, D.M., T. Tetsuka, D. Daphna-Iken, S. Srivastava, and A.R. Morrison. 1996. Interleukin-1β activates protein kinase C ζ in renal mesangial cells. J. Biol. Chem. 271: 17241-17246.
29. Schönwasser, D.C., R.M. Marais, C.J. Marshall, and P.J. Parker. 1998. Activation of the mitogen-activated protein kinase/extracellular signal-regulated kinase pathway by conventional, novel, and atypical protein kinase C isotypes. Mol. Cell. Biol. 18: 790-798.
30. Sontag, E., J.M. Sontag, and A. Garcia. 1997. Protein phosphatase 2A is a critical regulator of protein kinase C ζ signaling targeted by SV40 small t to promote cell growth and NF-κB activation. EMBO J. 16: 5662-5671.
31. Stancovski, I., and D. Baltimore. 1997. NF-κB activation: The IκB kinase revealed? Cell 91: 299-302.
32. Takeda, H., T. Matozaki, T. Takada, T. Noguchi, T. Yamao, M. Tsuda, F. Ochi, K. Fukunaga, K. Inagaki, and M. Kasuga. 1999. PI 3-kinase gamma and protein kinase C-ζ mediate RAS-independent activation of MAP kinase by a Gi protein-coupled receptor. EMBO J 18:386-395.
33. Thanos, D., and T. Maniatis. 1995. NF-κB: a lesson in family values. Cell 80: 529-532.
34. Verma, I.M., J.K. Stevenson, E.M. Schwarz, D. VanAntwerp, and S. Miyamoto. 1995. Rel/NF-κB/IκB family: intimate tales of association and dissociation. Genes Dev. 9: 2723-2735.
35. Woronicz, J.D., X. Gao, Z. Cao, M. Rothe and D.V. Goeddel. 1997. IκB kinase-β: NF-KB activation and complex formation with IκB kinase-α and NIK. Science 278: 866-869.
36. Zandi, E., D.M. Rothwarf, M. Delhase, M. Hayakawa, and M. Karin. 1997. The IκB kinase complex (IKK) contains two kinase subunits, IKKα and IKKβ, necessary for IκB phosphorylation and NF-κB activation. Cell 91: 243-252.

## Claims

1. An isolated human BLIP protein or a variant thereof.

2. An isolated protein having a sequence selected from:
(a) SEQ ID NO:4; and
(b) sequences that have at least about 70% sequence similarity to SEQ ID NO:4, and wherein the protein is a BLIP protein.

3. A kinase inactive mutant of a BLIP protein according to claim 2.

4. A kinase inactive mutant of a BLIP protein having SEQ ID NO:5.

5. An isolated protein having a sequence selected from:
(a) SEQ ID NO:6; and
(b) sequences that have at least about 90% sequence similarity to SEQ ID NO:6, and wherein the protein is a LIP protein.

6. An isolated nucleic acid molecule having a nucleotide sequence selected from:
(a) a nucleotide sequence encoding a protein of SEQ ID NO:4;
(b) a nucleotide sequence that hybridizes to the sequence of (a) above under conditions represented by a wash stringency of 0.3M NaCl, 0.03M sodium citrate, and 0.1 % SDS at 60 degrees C°, and which encodes a BLIP protein;
(c) a nucleotide sequence complementary to a sequence of (a) - (b), above.

7. An isolated nucleotide molecule according to claim 6 which is a cDNA sequence.

8. An isolated nucleic acid which encodes a BLIP protein having the amino acid sequence given herein as SEQ ID NO:4.

9. An isolated nucleic acid molecule having a nucleotide sequence selected from:
(a) a nucleotide sequence encoding a protein of SEQ ID NO:6;
(b) a nucleotide sequence that hybridizes to the sequence of (a) above under conditions represented by a wash stringency of 0.3M NaCl, 0.03M sodium citrate, and 0.1% SDS at 60 degrees C°, and which encodes a LIP protein with at least about 90% amino acid sequence similarity to SEQ ID NO:6;
(c) a nucleotide sequence complementary to a sequence of (a) - (b), above.

10. An isolated nucleic acid which encodes a LIP protein having the amino acid sequence given herein as SEQ ID NO:6.

11. A nucleic acid construct having a promoter and a heterologous nucleic acid operably linked to said promoter, wherein said heterologous nucleic acid is a nucleic acid according to claim 6-10.

12. A cell containing a nucleic acid construct according to claim 11.

13. A cell containing a nucleic acid construct according to claim 11 and capable of expressing the encoded protein.

14. An expression vector comprising a nucleotide sequence according to any one of claims 6 to 10, which is capable of expressing the encoded protein.

15. A cell line according to claim 13 which is a modified HEK293 or HeLa cell line.

16. A method of producing a BLIP protein comprising introducing into an appropriate cell line a suitable vector comprising a nucleotide sequence according to claim 6, under conditions suitable for obtaining expression of the nucleotide sequence.

17. An antibody that specifically binds to a protein encoded by a nucleic acid molecule of claim 6.

18. An antibody according to claim 17 that is a monoclonal antibody.

19. A method of screening compounds for the ability to modulate BLIP-dependent NFκB activation, comprising administering a test compound to a test cell and detecting any increase or decrease in BLIP-dependent NFκB activation in the test cell, compared to a control cell that was not administered the test compound.

20. A method according to claim 19 where said detection of BLIP-dependent NFκB activation utilizes a κB-dependent reporter gene.

21. A compound identified by the method according to claim 19.

22. A method of inhibiting NF-κB activation in a cell comprising administering to the cell an inhibitor of BLIP-related NF-κB activation, in an amount effective to inhibit NF-κB activation therein compared to the NF-κB activation that would occur in the absence of said inhibitor.

23. A method according to claim 22 wherein said inhibitor is a BLIP kinase-dead mutant or a LIP protein.

24. A method of treatment or prophylaxis of a disorder that is responsive to inhibition of BLIP-dependent NF-κB activation in a mammalian subject, comprising administering to said subject an effective amount of a compound that inhibits BLIP-dependent NF-κB activation.

25. A method according to claim 24 where said disorder is selected from neoplastic, inflammatory and immune disorders.

26. The use of a compound that modulates of BLIP-dependent NF-κB activation in a method of formulating a medicament for treatment or prophylaxis of a disorder that is responsive to modulation of BLIP-dependent NF-κB activation.
